# EUROPEAN PATENT APPLICATION

(11) **EP 3 967 692 A1**
(43) Date of publication of application: **16.03.2022**
(21) Application number: 20801783.0
(22) Date of filing: 08.05.2020
(51) Int. Cl.: C07D 471/04, A61K 31/519, A61P 35/00

(54) **CRYSTAL POLYMORPHISM OF PI3K INHIBITOR AND METHOD FOR PREPARING SAME**

(30) Priority: 09.05.2019 KR 20190054506
(71) Applicant: Boryung Pharmaceutical Co., Ltd., Seoul 03127 (KR)
(72) Inventor: KIM, Seong Heon, Seoul 07981 (KR); LEE, Joon Kwang, Gwangmyeong-si Gyeonggi-do 14293 (KR); SUN, Yong Ho, Gunpo-si Gyeonggi-do 15804 (KR); KIM, Ji Han, Seoul 07444 (KR)
(74) Representative: HGF
(86) International application number: PCT/IB2020/054366
(87) International publication number: WO 2020/225783

(57) **Abstract**

The present invention relates to a novel crystal polymorphism of a P13K inhibitor, and a method for preparing same. The novel crystal form of the present invention has remarkably superior non-hygroscopicity, can exhibit a superior pharmacological effect due to high blood levels, and exhibits high bioavailability, and thus is suitable for pharmaceutical formulations.

## Description

### [Technical Field]

The present invention relates to a crystal polymorphism of a novel P13K inhibitor defined as a specific powder X-ray diffraction pattern and a method for preparing the same.

### [Background Art]

Phosphatidylinositol 3-kinase (PI3 kinase; PI3K) is a lipid kinase, which phosphorylates lipid molecules instead of proteins, and plays an important role in cell survival, signal transduction, control of membrane trafficking, etc. If a problem occurs to such control, cancer, inflammatory diseases, autoimmune diseases, etc. occur.

Recently, study results for developing a compound having a novel structure effective in selectively inhibiting PI3 kinase have been reported, and specifically International Publication No. WO 2016/204429 discloses a compound which has PI3 kinase inhibitory activity and is useful for the treatment of cancer, autoimmune diseases, respiratory diseases and the like, which is incorporated herein by reference.

When designing a suitable dosage form in the process of preparing a drug, the physical state of a drug raw material of the drug, that is, whether the state is crystalline or amorphous, is very important.

However, a crystalline raw material has a problem in that solubility is low and bioavailability per unit weight is lowered.

### [Prior Art References]

### [Patent Documents]

(Patent Document 1) International Publication No. WO 2016/204429

### [Disclosure of Invention]

### [Technical Problem]

An object of the present invention is to provide a crystal polymorphism of a P13K inhibitor suitable for pharmaceutical preparations due to excellent non-hygroscopicity and stability and a high concentration in blood, and a method for preparing the same.

An object of the present invention is to provide a crystal form A of a compound represented by formula 1 below having a peak at diffraction angles (2θ±0.2°) of 6.71°, 7.36°, 9.39°, 12.34°, 20.76° and 24.13° in a powder X-ray diffraction pattern.

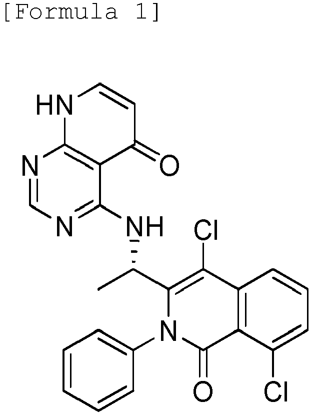

Other object of the present invention is to provide a method for preparing the crystal form A, the method including: 1) dissolving a compound represented by above formula 1 in C₁-C₄ alcohol; and 2) slowly cooling down the solution obtained from above step 1) to room temperature and stirring for 1 to 24 hours.

Another object of the present invention is to provide a crystal form B of a compound represented by formula 1 below having a peak at diffraction angles (2θ±0.2°) of 3.34°, 6.20°, 7.06°, 9.45°, 18.82° and 24.97° in a powder X-ray diffraction pattern.

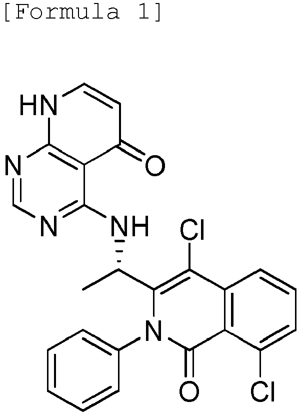

Another object of the present invention is to provide a method for preparing the crystal form B, the method including: 1) dissolving a compound represented by above formula 1 in acetone; and 2) slowly cooling down the solution obtained from above step 1) to room temperature and stirring for 1 to 24 hours.

Another object of the present invention is to provide a crystal form C of a compound represented by formula 1 below having a peak at diffraction angles (2θ±0.2°) of 3.72°, 6.27°, 7.22°, 9.58°, 18.90° and 25.14° in a powder X-ray diffraction pattern.

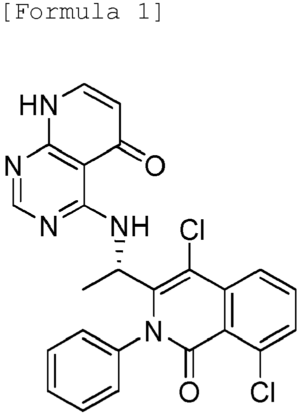

Another object of the present invention is to provide a method for preparing the crystal form C, the method including: 1) dissolving a compound represented by formula 1 according to claim 7 in C₁-C₄ alcohol; 2) adding water dropwise to the solution obtained from above step 1); and 3) slowly cooling down to room temperature and stirring for 1 to 24 hours.

Another object of the present invention is to provide a crystal form D of a compound represented by formula 1 below having a peak at diffraction angles (2θ±0.2°) of 3.77°, 6.14°, 6.88°, 7.51°, 9.25° and 9.92° in a powder X-ray diffraction pattern.

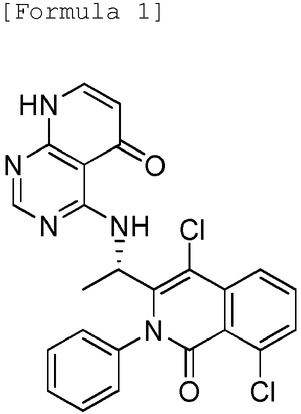

Another object of the present invention is to provide a method for preparing the crystal form D, the method including: 1) dissolving a compound represented by above formula 1 in acetonitrile; and 2) slowly cooling down the solution obtained from above step 1) to room temperature and stirring for 1 to 24 hours.

Another object of the present invention is to provide a crystal form E of a compound represented by formula 1 below having a peak at diffraction angles (2θ±0.2°) of 3.81°, 6.25°, 6.98°, 7.61°, 9.35° and 26.74° in a powder X-ray diffraction pattern.

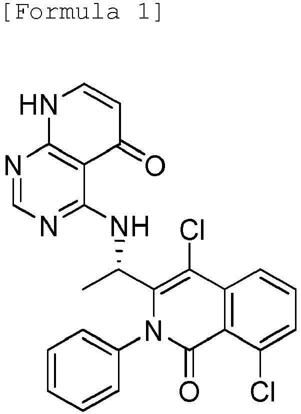

Another object of the present invention is to provide a method for preparing the crystal form E, the method including: 1) dissolving a compound represented by above formula 1 in acetonitrile; 2) adding water dropwise to the solution obtained from above step 1); and 3) slowly cooling down to room temperature and stirring for 1 to 24 hours.

Another object of the present invention is to provide a crystal form G of a compound represented by formula 1 below having a peak at diffraction angles (2θ±0.2°) of 7.33°, 8.56°, 9.69°, 9.94°, 19.97° and 25.37° in a powder X-ray diffraction pattern.

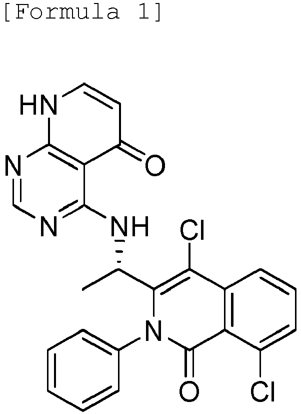

Another object of the present invention is to provide a method for preparing the crystal form G, the method including: 1) dissolving a compound represented by above formula 1 in 1,4-dioxane; and 2) slowly cooling down the solution obtained from above step 1) to room temperature and stirring for 1 to 24 hours.

Another object of the present invention is to provide a crystal form H of a compound represented by formula 1 below having a peak at diffraction angles (2θ±0.2°) of 7.48°, 10.24°, 10.35°, 11.10°, 13.55° and 15.18° in a powder X-ray diffraction pattern.

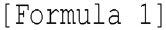

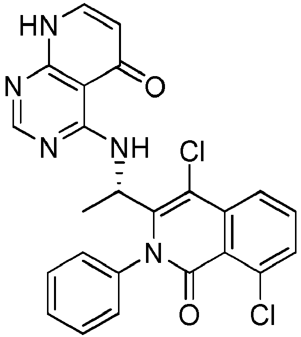

Another object of the present invention is to provide a method for preparing the crystal form H, the method including: 1) dissolving a compound represented by above formula 1 in tetrahydrofuran; and 2) slowly cooling down the solution obtained from above step 1) to room temperature and stirring for 1 to 24 hours.

Another object of the present invention is to provide a crystal form I of a compound represented by formula 1 below having a peak at diffraction angles (2θ±0.2°) of 12.03°, 15.19°, 21.77°, 22.81°, 24.21° and 27.11° in a powder X-ray diffraction pattern.

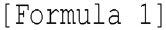

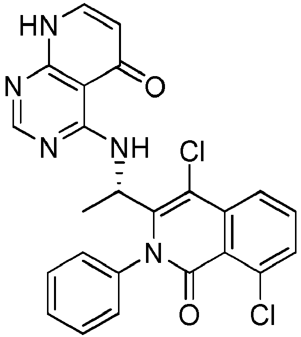

Another object of the present invention is to provide a method for preparing the crystal form I, the method including: 1) adding a compound represented by above formula 1 into water, followed by refluxing; and 2) slowly cooling down the resulting solution to room temperature and stirring for 1 to 24 hours.

### [Technical Solution]

Accordingly, the present inventors have made efforts to find an long-term stable and industrially applicable form of (S)-4-((1-(4,8-dichloro-1-oxo-2-phenyl-1,2-dihydroisoquinoline-3-yl)ethyl)amino)pyrido[2,3-d]pyrimidin-5(8H)-one (hereinafter, the compound of formula 1), which is one of heteroaryl derivatives known to have PI3 kinase inhibitory activity, so that this form may be used as a pharmaceutical preparation. In result, it has confirmed that a crystal polymorphism of the compound having a specific powder X-ray diffraction pattern is advantageous for being formulated into preparations due to low hygroscopicity, is excellent in stability depending on accelerated, long-term and stress storage conditions, thermal stability and stability depending on pH, and thus may be stably maintained without any change in the content in the long term, and a crystal polymorphism of the compound shows an excellent pharmacological effect with a high concentration in blood and shows high bioavailability leading to an excellent therapeutic effect even with a small amount of use, thereby completing the present invention.

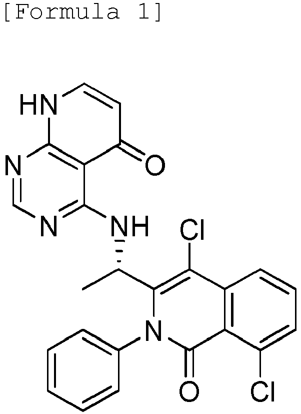

The present invention provides a crystal polymorphism of a P13K inhibitor and a method for preparing the same.

A crystal form may have an advantage of securing stability and making a continuously controlled release dosage form, and thus most pharmaceutical dosage forms may use crystalline raw materials, except for special cases.

The crystal polymorphism of the P13K inhibitor of the present invention may have high purity, may be safe, and may have excellent stability against heat and humidity, and thus may be stably maintained without any change in content in the long term. Thus, the same state may be maintained even after producing a preparation by using the crystal polymorphism of the PI3K inhibitor of the present invention or producing a preparation containing the same, and thus the content uniformity of the preparation may be stably maintained for a long period of time and excellent stability may be maintained for a long period of time due to remarkably less occurrence of impurities. In addition, the crystal polymorphism of the PI3K inhibitor may be easily applied to mass production, and the polymorphism of the compound may exhibit non-hygroscopicity, and thus may be very advantageous for processing and storage of pharmaceuticals, and may be easily formulated into preparations.

The crystal polymorphism of the P13K inhibitor of the present invention may exhibit an excellent pharmacological effect by showing a high concentration in blood, and thus may be effectively used as a novel effective ingredient in a pharmaceutical composition capable of treating cancer diseases.

The crystal polymorphism of the P13K inhibitor of the present invention may show high bioavailability when orally administered, and thus may exhibit an excellent therapeutic effect even with a small amount of use, thereby remarkably improving the convenience of taking medication for patients. In addition, the crystal polymorphism of the P13K inhibitor of the present invention may have a rapid onset of action and a thermodynamically stable form.

Furthermore, in the case of the method for preparing the crystal polymorphism of the P13K inhibitor of the present invention, mass production may be possible with high yield and purity at low cost.

In the present specification, the term "crystal polymorphism" means one in which arrangement of constituent particles is regular and the constituent units of a crystal are the same only with a different state of arrangement.

Hereinafter, the present invention will be described in more detail.

### Crystal form A

The present invention provides a crystal form A of a compound represented by formula 1 below having a peak at diffraction angles (2θ±0.2°) of 6.71°, 7.36°, 9.39°, 12.34°, 20.76° and 24.13° in a powder X-ray diffraction pattern.

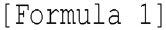

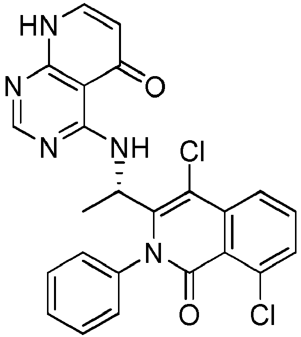

The crystal form A of the present invention may have high purity, may be safe, and may have excellent stability against heat and humidity, and thus may be stably maintained without any change in content in the long term. Thus, the same state may be maintained even after producing a preparation by using the crystal form A of the present invention or producing a preparation containing the same, and thus the content uniformity of the preparation may be stably maintained for a long period of time and may be easily applied to mass production. In addition, the crystal form A of the present invention may exhibit a high concentration in blood and thus have high bioavailability, thereby exhibiting an excellent pharmacological effect. Thus, the crystal form A of the present invention may be effectively used as a novel effective ingredient in a pharmaceutical composition capable of treating cancer diseases, and the crystal form A of the present invention may show high bioavailability when orally administered, and thus may exhibit an excellent therapeutic effect even with a small amount of use, so that the form may be safe, have fewer side effects, and remarkably improve the convenience of taking medication for patients.

In one embodiment of the present invention, the crystal form A of the compound represented by above formula 1 may further has at least one peak selected from diffraction angles (2θ±0.2°) of 5.75°, 8.19°, 9.87°, 11.12°, 13.71°, 16.12°, 17.88°, 18.90°, 19.71°, 23.21°, 25.18° and 27.09° in a powder X-ray diffraction pattern.

In one embodiment of the present invention, the crystal form A of the compound represented by above formula 1 may be one in which a powder X-ray diffraction pattern is obtained by using CuKα radiation.

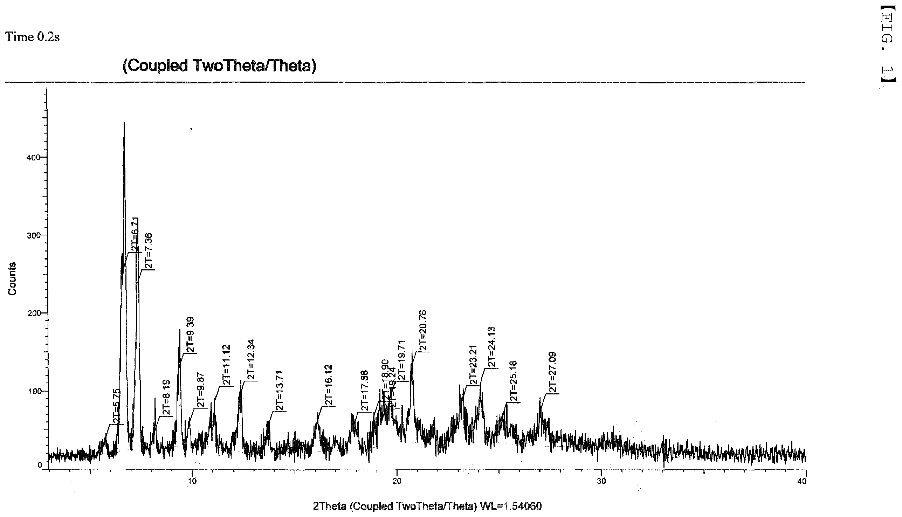

The crystal form A of the compound represented by above formula 1 of the present invention may have substantially the same spectrum as a powder X-ray diffraction (PXRD) analysis spectrum shown in FIG. 1.

The compound represented by above formula 1 may selectively inhibit PI3 kinase selected from the group consisting of PI3Kα, PI3Kβ, PI3Kδ and PI3Kγ.

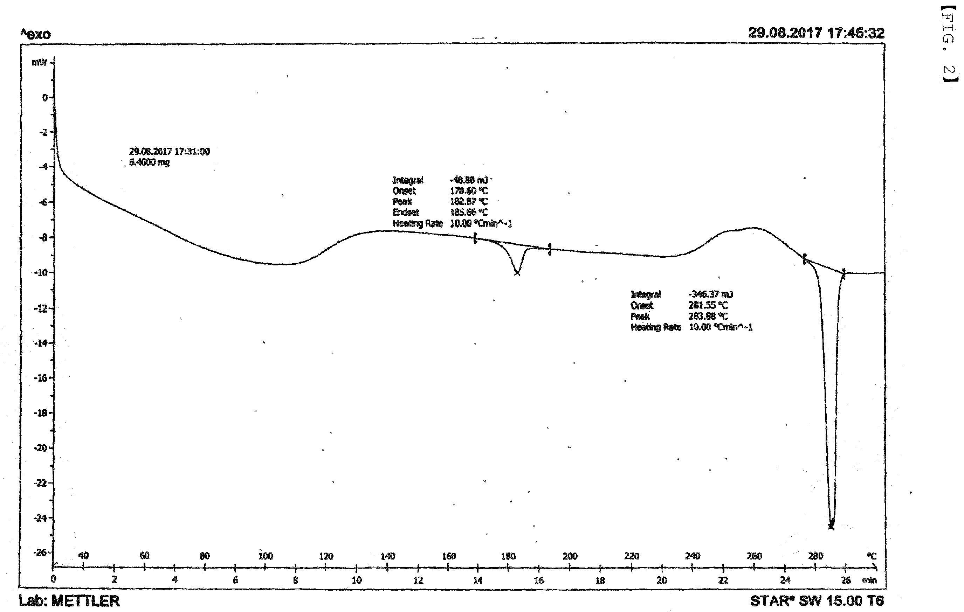

The crystal form A of the compound represented by above formula 1 may exhibit a differential scanning calorimetry (DSC) endothermic transition peak at 178 to 185°C when a heating rate is 10°C/min. Specifically, the crystal form A may have a DSC spectrum as shown in FIG. 2.

In one embodiment of the present invention, the crystal form A of the compound represented by formula 1 may be anhydride as a non-solvate.

### Method for preparing crystal form A

The present invention provides a method for preparing the crystal form A, the method including: 1) dissolving a compound represented by above formula 1 in C₁-C₄ alcohol; and 2) slowly cooling down the solution obtained from above step 1) to room temperature and stirring for 1 to 24 hours.

In the case of the method for preparing the crystal form A of the present invention, mass production may be possible with high yield and purity at low cost.

In the present invention, in the expression "Cx" of a functional group, x represents the number of carbons, and Cx to Cy means a functional group having x or more and y or less in the number of carbons.

In the present invention, the term "alcohol" means a compound in which a hydroxyl group is bonded to a carbon atom of an alkyl or substituted alkyl group, and specifically the alcohols means an alcohol in which a hydroxyl group is bonded to a carbon atom of a linear or branched C₁-C₄ alkyl group or a substituted alkyl group. One example of C₁-C₄ alcohol may include methanol, ethanol, propanol, isopropanol, butanol, etc., and specifically ethanol may be used as a solvent in above step 1).

In the present specification, the term "alkyl group" means a linear saturated hydrocarbon group or a branched saturated hydrocarbon group, and the term "substituted alkyl group" means one in which a substituent bonded with carbon of an alkyl group is substituted with hydroxy, cyano, halo, etc.

In one embodiment of the present invention, above step 1) may be a step of stirring under reflux. Specifically, in the present specification, the term "stirring under reflux" means stirring under reflux conditions, and the term "reflux conditions" means conditions in which liquid boils and the vapor of this boiling liquid is condensed and returned to the bottom of the liquid to work therein. Specifically, the term means one in which, in step 1), the vapor of C₁-C₄ alcohol is condensed and returned to the bottom of the liquid to dissolve the compound represented by formula 1 by stirring.

In one embodiment of the present invention, above step 2) may include a step of slowly cooling down the solution obtained from step 1) to room temperature. Specifically, in the step of cooling, rapid cooling may cause a phase transition, etc., of the resulting crystal form A, and thus cooling may be slowly performed at a constant rate as much as possible, for example, at a rate of 0.1°C to 10°C per minute, and methods such as natural cooling, etc., may be also used. The natural cooling may be one in which the heat source used for heat treatment is simply removed. As such, a sufficient cooling rate may be obtained only by removing the heat source as described above.

In the present invention, the term "room temperature" may be a natural temperature which is not heated or lowered, and may mean a temperature of about 10°C to 30°C, about 25°C, or about 23°C.

The method for preparing the crystal form A may further include step 3) below.

In one embodiment of the present invention, step 3) may be a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be ethanol.

The method for preparing the crystal form A may further include step 4) below.

In one embodiment of the present invention, step 4) may be a step of drying the washed solid obtained from step 3) at a temperature of 30 to 60°C, specifically hot air drying at a temperature of 35 to 55°C.

### Crystal form B

The present invention provides a crystal form B of a compound represented by above formula 1 having a peak at diffraction angles (2θ±0.2°) of 3.34°, 6.20°, 7.06°, 9.45°, 18.82° and 24.97° in a powder X-ray diffraction analysis pattern.

The crystal form B of the present invention may have high purity, may be safe, and may have excellent stability against heat and humidity, and thus may be stably maintained without any change in content in the long term. Thus, the same state may be maintained even after producing a preparation by using the crystal form B of the present invention or producing a preparation containing the same, and thus the content uniformity of the preparation may be stably maintained for a long period of time and may be easily applied to mass production. In addition, the crystal form B of the present invention may exhibit an excellent pharmacological effect due to a high concentration in blood and high bioavailability. Thus, the crystal form B of the present invention may be effectively used as a novel effective ingredient in a pharmaceutical composition capable of treating cancer diseases, and the crystal form B of the present invention may show high bioavailability when orally administered, and thus may exhibit an excellent therapeutic effect even with a small amount of use, so that the form may be safe, have fewer side effects, and remarkably improve the convenience of taking medication for patients.

In one embodiment of the present invention, the crystal form B of the compound represented by above formula 1 may further has at least one peak at diffraction angles (2θ±0.2°) of 7.33°, 8.06°, 8.25°, 13.89°, 16.22° and 26.42° in a powder X-ray diffraction analysis pattern.

In one embodiment of the present invention, the crystal form B of the compound represented by above formula 1 may be one in which a powder X-ray diffraction analysis pattern is obtained by using CuKαradiation.

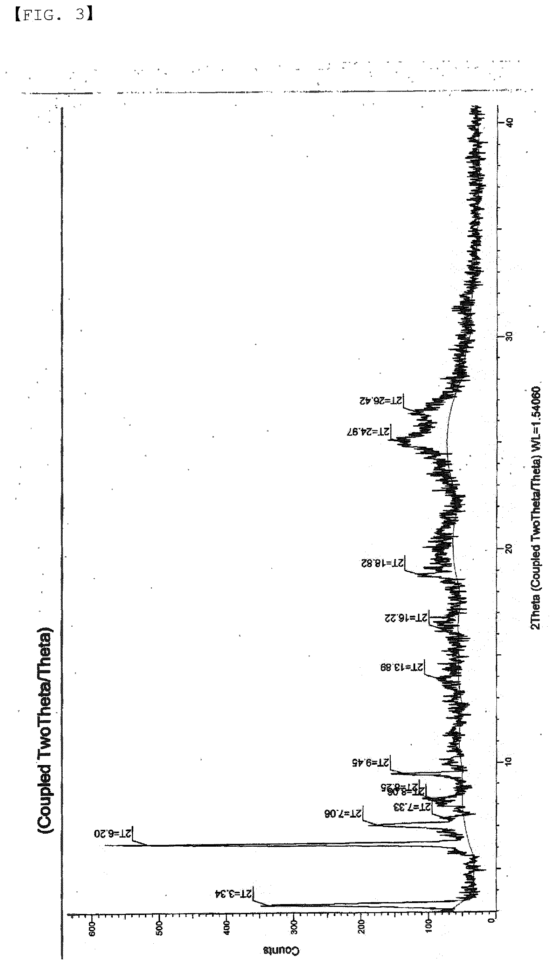

The crystal form B of the compound represented by above formula 1 of the present invention may have substantially the same spectrum as a powder X-ray diffraction (PXRD) analysis spectrum shown in FIG. 3.

The compound represented by above formula 1 may selectively inhibit PI3 kinase selected from the group consisting of PI3Kα, PI3Kβ, PI3Kδ and PI3Kγ.

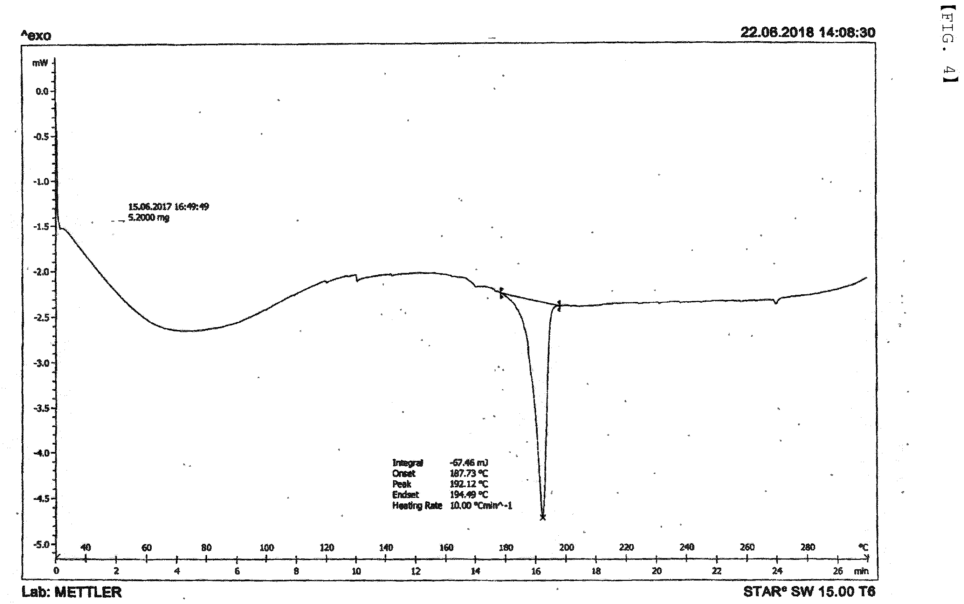

The crystal form B of the compound represented by above formula 1 may exhibit a differential scanning calorimetry (DSC) endothermic transition peak at 187 to 194°C when a heating rate is 10°C/min. Specifically, the crystal form B may have a DSC spectrum as shown in FIG. 4.

In one embodiment of the present invention, the crystal form B of the compound represented by formula 1 may be anhydride as a non-solvate.

### Method for preparing crystal form B

The present invention may provide a method for preparing the crystal form B, the method including: 1) dissolving a compound represented by above formula 1 in C₃-C₁₀ ketone; and 2) slowly cooling down the solution obtained from above step 1) to room temperature and stirring for 1 to 24 hours.

In the case of the method for preparing the crystal form B of the present invention, mass production may be possible with high yield and purity at low cost.

In the present specification, the term "ketone" may be a kind of organic solvent, and the number of carbon atoms in a main chain of ketone may be C₃-C₁₀, in which the main chain may be linear or branched, and one example of ketone may include acetone, methyl ethyl ketone, methyl butyl ketone, methyl isobutyl ketone, etc. Specifically, acetone may be used as a solvent in above step 1).

Above step 1) may be a step of stirring under reflux. In this regard, the content may be the same as described in the method for preparing the crystal form A.

The method for preparing the crystal form B may further include step 3) below.

In one embodiment of the present invention, step 3) may further include a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be acetone.

In the present invention, the description about the term "room temperature" may be the same as described in the method for preparing the crystal form A.

The method for preparing the crystal form B may further include step 4) below.

In one embodiment of the present invention, step 4) may be a step of drying the washed solid obtained from step 3) at a temperature of 30 to 60°C, specifically hot air drying at a temperature of 35 to 55°C.

### Crystal form C

The present invention may provide a crystal form C of a compound represented by above formula 1 having a peak at diffraction angles (2θ±0.2°) of 3.72°, 6.27°, 7.22°, 9.58°, 18.90° and 25.14° in a powder X-ray diffraction analysis pattern.

The crystal form C of the present invention may have high purity, may be safe, and may have excellent stability against heat and humidity, and thus may be stably maintained without any change in content in the long term. Thus, the same state may be maintained even after producing a preparation by using the crystal form C of the present invention or producing a preparation containing the same, and thus the content uniformity of the preparation may be stably maintained for a long period of time and may be easily applied to mass production. In addition, the crystal form C of the present invention may exhibit a high concentration in blood and thus have high bioavailability, thereby exhibiting an excellent pharmacological effect. Thus, the crystal form C of the present invention may be effectively used as a novel effective ingredient in a pharmaceutical composition capable of treating cancer diseases, and the crystal form C of the present invention may show high bioavailability when orally administered, and thus may exhibit an excellent therapeutic effect even with a small amount of use, so that the form may be safe, have fewer side effects, and remarkably improve the convenience of taking medication for patients.

In one embodiment of the present invention, the crystal form C of the compound represented by above formula 1 may further include at least one peak at diffraction angles (2θ±0.2°) of 5.11°, 7.42°, 8.09°, 10.15°, 13.08°, 16.37°, and 26.53° and 31.43° in a powder X-ray diffraction analysis pattern.

In one embodiment of the present invention, the crystal form C of the compound represented by above formula 1 may be one in which a powder X-ray diffraction analysis pattern is obtained by using CuKα radiation.

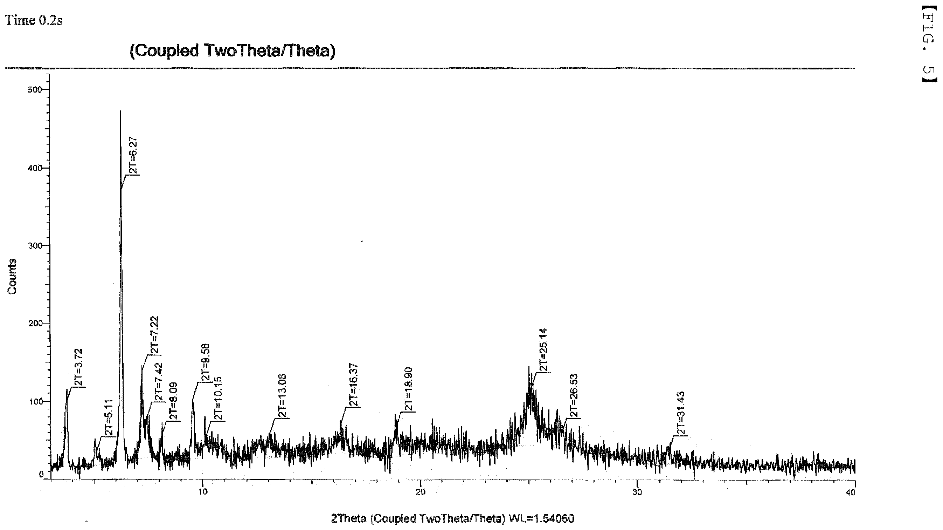

The crystal form C of the compound represented by above formula 1 of the present invention may have substantially the same spectrum as a powder X-ray diffraction (PXRD) analysis spectrum shown in FIG. 5.

The compound represented by above formula 1 may selectively inhibit PI3 kinase selected from the group consisting of PI3Kα, PI3Kβ, PI3Kδ and PI3Kγ.

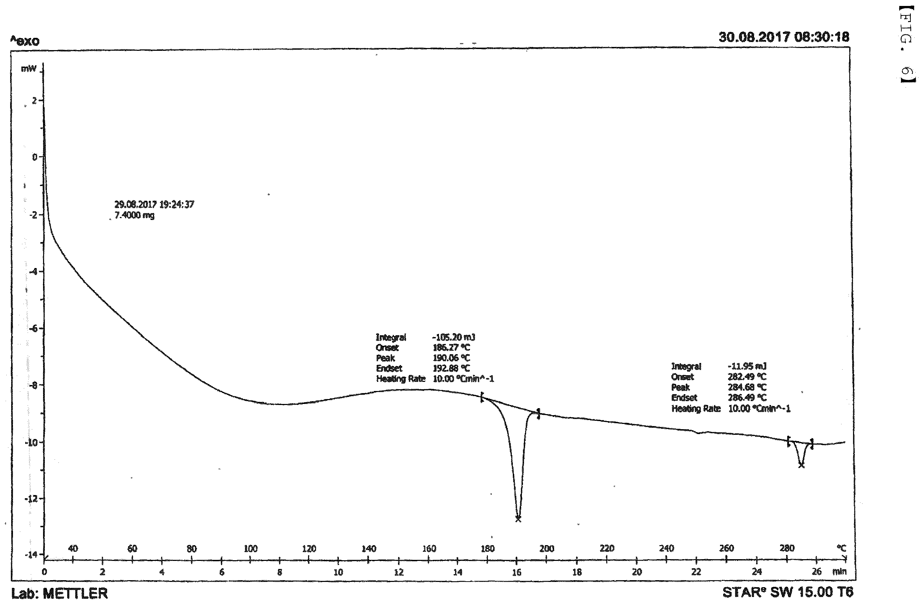

The crystal form C of the compound represented by above formula 1 may exhibit a differential scanning calorimetry (DSC) endothermic transition peak at 186 to 192°C when a heating rate is 10°C/min. Specifically, the crystal form C may have a DSC spectrum as shown in FIG. 6.

In one embodiment of the present invention, the crystal form C of the compound represented by formula 1 may be anhydride as a non-solvate.

### Method for preparing crystal form C

Another aspect of the present invention is to provide a method for preparing the crystal form C, the method including: 1) dissolving a compound represented by above formula 1 in C₁-C₄ alcohol; 2) adding water dropwise to the solution obtained from above step 1); and 3) slowly cooling down to room temperature and stirring for 1 to 24 hours.

In the case of the method for preparing the crystal form C of the present invention, mass production may be possible with high yield and purity at low cost.

The content of the C₁-C₄ alcohol may be the same as described in the method for preparing the crystal form A, and specifically ethanol may be used as a solvent in above step 1).

In one embodiment of the present invention, above step 1) may be a step of stirring under reflux. In this regard, the content may be the same as described in the method for preparing the crystal form A.

In one embodiment of the present invention, in above step 2), water may be added dropwise to the solution obtained from step 1), in which the solution obtained from step 1) and water may be mixed at a volume ratio of 1-2: 1-2.

In one embodiment of the present invention, above step 3) may include a step of slowly cooling down the solution obtained from step 2) to room temperature. In this regard, the content may be the same as described in the method for preparing the crystal form A.

The method for preparing the crystal form C may further include step 4) below.

In one embodiment of the present invention, step 4) may further include a step of filtering a solid obtained from step 3) and washing with any one solvent selected from the group consisting of ethanol, acetone, acetonitrile, 1,4-dioxane, tetrahydrofuran, water and a mixture thereof. Specifically, the solvent used in above step 4) may be ethanol.

In the present invention, the description about the term "room temperature" may be the same as described in the method for preparing the crystal form A.

The method for preparing the crystal form C may further include step 5) below.

In one embodiment of the present invention, step 5) may be a step of drying the washed solid obtained from step 4) at a temperature of 30 to 60°C, specifically hot air drying at a temperature of 35 to 55°C.

### Crystal form D

The present invention may provide a crystal form D of a compound represented by above formula 1 having a peak at diffraction angles (2θ±0.2°) of 3.77°, 6.14°, 6.88°, 7.51°, 9.25° and 9.92° in a powder X-ray diffraction analysis pattern.

The crystal form D of the present invention may have high purity, may be safe, and may have excellent stability against heat and humidity, and thus may be stably maintained without any change in content in the long term. Thus, the same state may be maintained even after producing a preparation by using the crystal form D of the present invention or producing a preparation containing the same, and thus the content uniformity of the preparation may be stably maintained for a long period of time and may be easily applied to mass production. In addition, the crystal form D of the present invention may exhibit a high concentration in blood and thus have high bioavailability, thereby exhibiting an excellent pharmacological effect. Thus, the crystal form D of the present invention may be effectively used as a novel effective ingredient in a pharmaceutical composition capable of treating cancer diseases, and the crystal form D of the present invention may show high bioavailability when orally administered, and thus may exhibit an excellent therapeutic effect even with a small amount of use, so that the form may be safe, have fewer side effects, and remarkably improve the convenience of taking medication for patients.

In one embodiment of the present invention, the crystal form D of the compound represented by above formula 1 may further include at least one peak at diffraction angles (2θ±0.2°) of 10.57°, 12.37°, 12.69°, 14.09°, 14.79°, 15.75°, 17.18°, 18.13°, 18.85°, 19.61°, 20.22°, 20.99°, 23.39°, 23.87°, 24.61°, 26.42°, 27.63°, 29.76° and 30.31° in a powder X-ray diffraction analysis pattern.

In one embodiment of the present invention, the crystal form D of the compound represented by above formula 1 may be one in which a powder X-ray diffraction analysis pattern is obtained by using CuKα radiation.

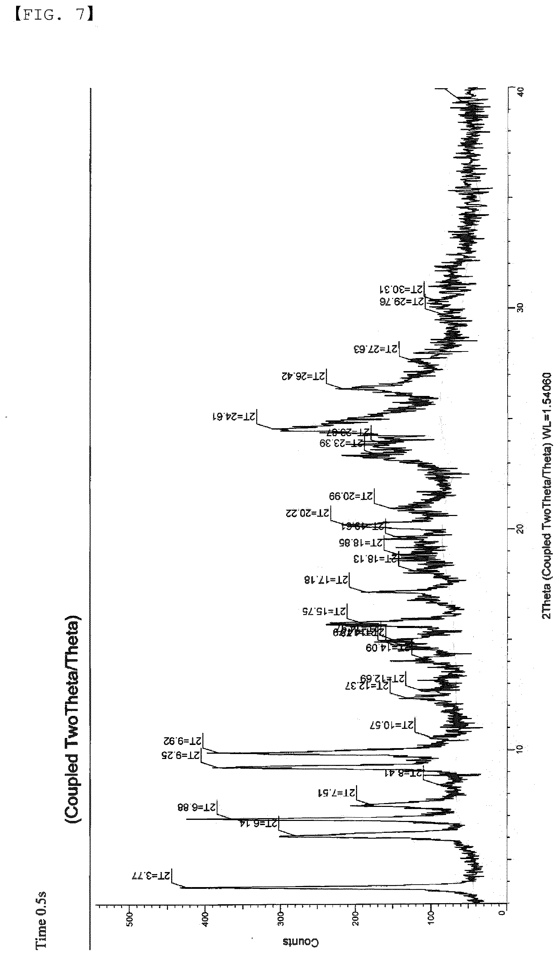

The crystal form D of the compound represented by above formula 1 of the present invention may have substantially the same spectrum as a powder X-ray diffraction (PXRD) analysis spectrum shown in FIG. 7.

The compound represented by above formula 1 may selectively inhibit PI3 kinase selected from the group consisting of PI3Kα, PI3Kβ, PI3Kδ and PI3Kγ.

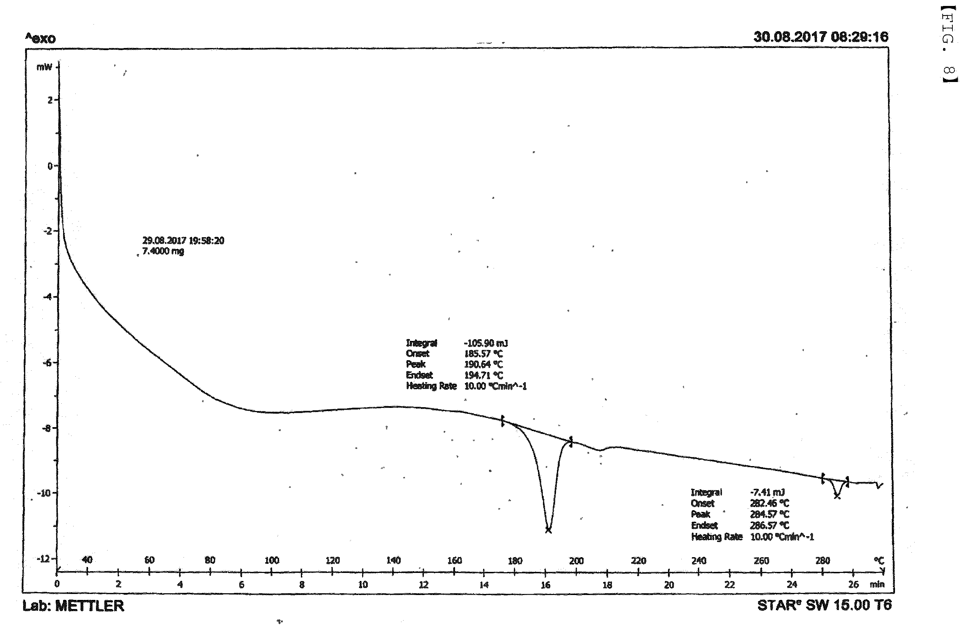

The crystal form D of the compound represented by above formula 1 may exhibit a differential scanning calorimetry (DSC) endothermic transition peak at 185 to 194°C when a heating rate is 10°C/min. Specifically, the crystal form D may have a DSC spectrum as shown in FIG. 8.

In one embodiment of the present invention, the crystal form D of the compound represented by formula 1 may be anhydride as a non-solvate.

### Method for preparing crystal form D

Another aspect of the present invention may provide a method for preparing the crystal form D, the method including: 1) dissolving a compound represented by above formula 1 in C₁-C₆ nitrile; and 2) slowly cooling down the solution obtained from above step 1) to room temperature and stirring for 1 to 24 hours.

In the case of the method for preparing the crystal form D of the present invention, mass production may be possible with high yield and purity at low cost.

In the present specification, the term "nitrile" may be represented by CN, and C₁-C₆ nitrile means an organic solvent with high volatility. One example of nitrile may include acetonitrile, propionitrile, butyronitrile, etc. Specifically, acetonitrile may be used as a solvent in above step 1).

In one embodiment of the present invention, above step 1) may be a step of stirring under reflux. In this regard, the content may be the same as described in the method for preparing the crystal form A.

In one embodiment of the present invention, above step 2) may include a step of slowly cooling down the solution obtained from step 1) to room temperature. In this regard, the content may be the same as described in the method for preparing the crystal form A.

The method for preparing the crystal form D may further include step 3) below.

In one embodiment of the present invention, step 3) may further include a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be acetonitrile.

In the present invention, the description about the term "room temperature" may be the same as described in the method for preparing the crystal form A.

The method for preparing the crystal form D may further include step 4) below.

In one embodiment of the present invention, step 4) may be a step of drying the washed solid obtained from step 3) at a temperature of 30 to 60°C, specifically hot air drying at a temperature of 35 to 55°C.

### Crystal form E

The present invention may provide a crystal form E of a compound represented by above formula 1 having a peak at diffraction angles (2θ±0.2°) of 3.81°, 6.25°, 6.98°, 7.61°, 9.35° and 26.74° in a powder X-ray diffraction analysis pattern.

The crystal form E of the present invention may have high purity, may be safe, and may have excellent stability against heat and humidity, and thus may be stably maintained without any change in content in the long term. Thus, the same state may be maintained even after producing a preparation by using the crystal form E of the present invention or producing a preparation containing the same, and thus the content uniformity of the preparation may be stably maintained for a long period of time and may be easily applied to mass production. In addition, the crystal form E of the present invention may exhibit a high concentration in blood and thus have high bioavailability, thereby exhibiting an excellent pharmacological effect. The crystal form E of the present invention may be effectively used as a novel effective ingredient in a pharmaceutical composition capable of treating cancer diseases, and the crystal form E of the present invention may show high bioavailability when orally administered, and thus may exhibit an excellent therapeutic effect even with a small amount of use, so that the form may be safe, have fewer side effects, and remarkably improve the convenience of taking medication for patients.

In one embodiment of the present invention, the crystal form E of the compound represented by above formula 1 may further include at least one peak at diffraction angles (2θ±0.2°) of 10.29°, 10.67°, 12.04°, 12.36°, 13.51°, 14.28°, 14.76°, 15.16°, 15.87°, 16.28°, 16.98°, 17.89°, 18.71°, 19.47°, 19.77°, 20.66°, 21.07°, 22.56°, 23.04°, 23.56°, 24.98°, 25.39°, 25.77°, 27.11°, 28.08°, 28.34° and 29.24° in a powder X-ray diffraction analysis pattern.

In one embodiment of the present invention, the crystal form E of the compound represented by above formula 1 may be one in which a powder X-ray diffraction analysis pattern is obtained by using CuKα radiation.

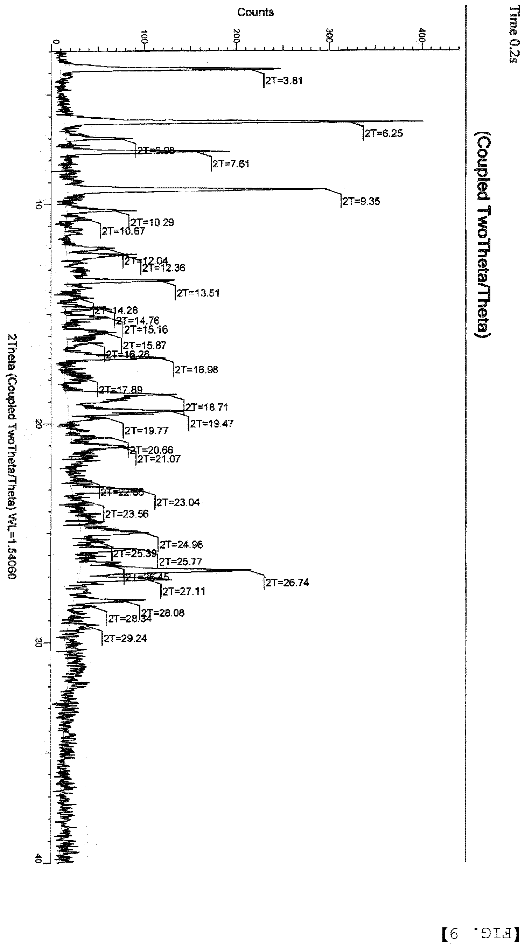

The crystal form E of the compound represented by above formula 1 of the present invention may have substantially the same spectrum as a powder X-ray diffraction (PXRD) analysis spectrum shown in FIG. 9.

The compound represented by above formula 1 may selectively inhibit PI3 kinase selected from the group consisting of PI3Kα, PI3Kβ, PI3Kδ and PI3Kγ.

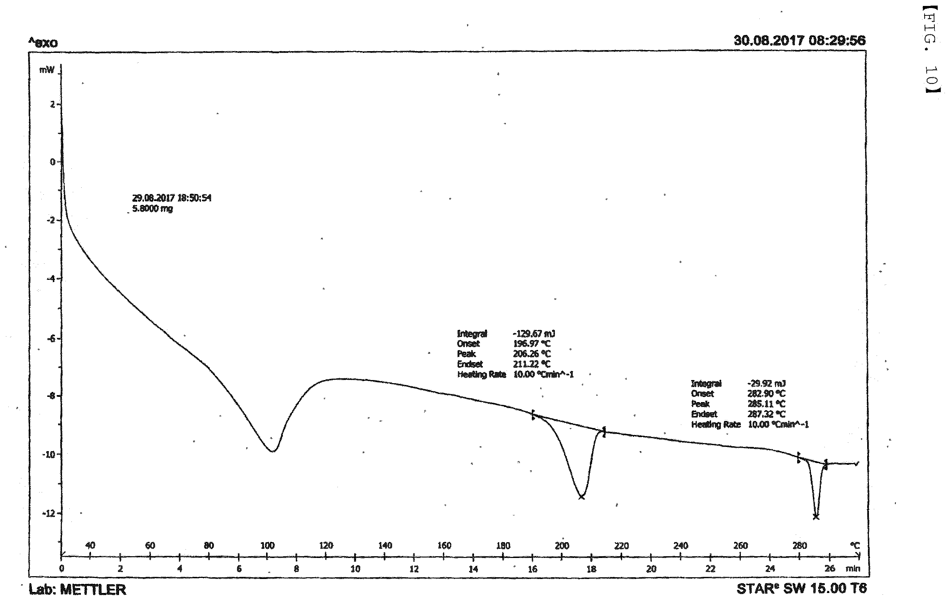

The crystal form E of the compound represented by above formula 1 may exhibit a differential scanning calorimetry (DSC) endothermic transition peak at 196 to 211°C when a heating rate is 10°C/min. Specifically, the crystal form E may have a DSC spectrum as shown in FIG. 10.

In one embodiment of the present invention, the crystal form E of the compound represented by formula 1 may be anhydride as a non-solvate.

### Method for preparing crystal form E

The present invention may provide a method for preparing the crystal form E, the method including: 1) dissolving a compound represented by above formula 1 in C₁-C₆ nitrile; 2) adding water dropwise to the solution obtained from above step 1); and 3) stirring at room temperature for 1 to 24 hours.

In the case of the method for preparing the crystal form E of the present invention, mass production may be possible with high yield and purity at low cost.

The content of the C₁-C₆ nitrile may be the same as described in the method for preparing the crystal form D, and specifically acetonitrile may be used as a solvent in above step 1), but is not limited thereto.

In one embodiment of the present invention, above step 1) may be a step of stirring under reflux. In this regard, the content may be the same as described in the method for preparing the crystal form A.

In one embodiment of the present invention, in above step 2), water may be added dropwise to the solution obtained from step 1), in which the solution obtained from step 1) and water may be mixed at a volume ratio of 1-2 : 1-2, but is not limited thereto.

The method for preparing the crystal form E may further include step 4) below.

In one embodiment of the present invention, step 4) may further include a step of filtering a solid obtained from step 3) and washing with any one solvent selected from the group consisting of ethanol, acetone, acetonitrile, 1,4-dioxane, tetrahydrofuran, water and a mixture thereof. Specifically, the solvent used in above step 4) may be acetonitrile.

In the present invention, the description about the term "room temperature" may be the same as described in the method for preparing the crystal form A.

The method for preparing the crystal form E may further include step 5) below.

In one embodiment of the present invention, step 5) may be a step of drying the washed solid obtained from step 4) at a temperature of 30 to 60°C, specifically hot air drying at a temperature of 35 to 55°C.

### Crystal form G

The present invention may provide a crystal form G of a compound represented by above formula 1 having a peak at diffraction angles (2θ±0.2°) of 7.33°, 8.56°, 9.69°, 9.94°, 19.97° and 25.37° in a powder X-ray diffraction analysis pattern.

In one embodiment of the present invention, the crystal form G of the compound represented by above formula 1 may be a 1,4-dioxane solvate of the compound represented by above formula 1. Specifically, the crystal form G may be a 1,4-dioxane solvate of the compound represented by above formula 1, and more specifically in a solvate of the compound represented by above formula 1, which is the crystal form G, a molar ratio between the compound represented by above formula 1 and 1,4-dioxane may be 1:1 to 3, specifically 1:1.

The crystal form G, which is a 1,4-dioxane solvate of the compound represented by above formula 1 of the present invention, may have high purity, may be safe, and may have excellent stability against heat and humidity, and thus may be stably maintained without any change in content in the long term. Thus, the same state may be maintained even after producing a preparation by using the crystal form G, which is a 1,4-dioxane solvate of the compound represented by above formula 1 of the present invention, or producing a preparation containing the same, and thus the content uniformity of the preparation may be stably maintained for a long period of time and may be easily applied to mass production.

In addition, the crystal form G, which is a 1,4-dioxane solvate of the compound represented by above formula 1 of the present invention, may exhibit non-hygroscopicity, and thus may be very advantageous for processing and storage of pharmaceuticals, and thus may be easily formulated into a preparation.

Furthermore, the crystal form G, which is a 1,4-dioxane solvate of the compound represented by above formula 1 of the present invention, may exhibit a high concentration in blood and thus have high bioavailability, thereby exhibiting an excellent pharmacological effect.

Thus, the crystal form G, which is a 1,4-dioxane solvate of the compound represented by above formula 1 of the present invention, may be effectively used as a novel effective ingredient in a pharmaceutical composition capable of treating cancer diseases.

In one embodiment of the present invention, the crystal form G of the compound represented by above formula 1 may further include at least one peak at diffraction angles (2θ±0.2°) of 4.94°, 13.34° and 15.53° in a powder X-ray diffraction analysis pattern.

In one embodiment of the present invention, the crystal form G of the compound represented by above formula 1 may be one in which a powder X-ray diffraction analysis pattern is obtained by using CuKα radiation.

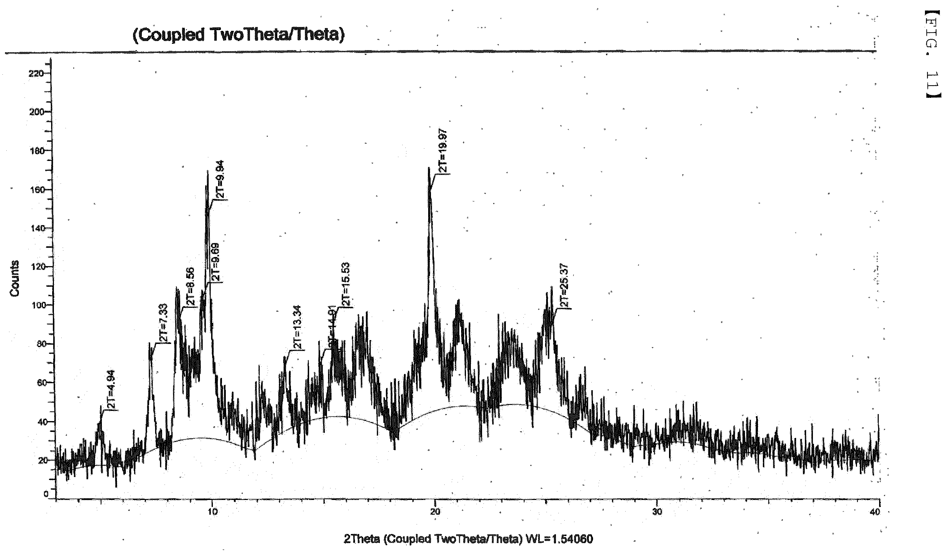

The crystal form G, which is a 1,4-dioxane solvate of the compound represented by above formula 1 of the present invention, may have substantially the same spectrum as a powder X-ray diffraction (PXRD) analysis spectrum shown in FIG. 11.

The crystal form G, which is a 1,4-dioxane solvate of the compound represented by above formula 1, may selectively inhibit PI3 kinase selected from the group consisting of PI3Kα, PI3Kβ, PI3Kδ and PI3Kγ.

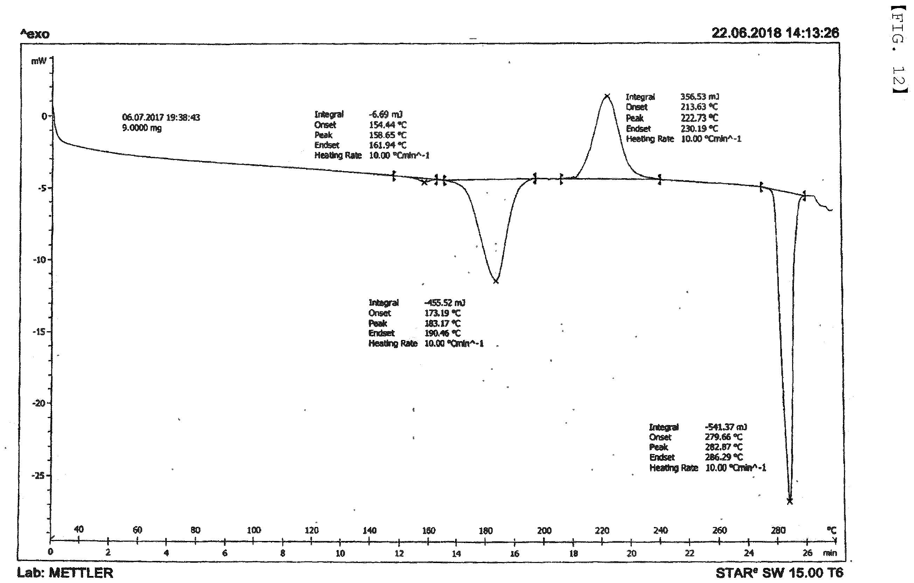

The crystal form G, which is a 1,4-dioxane solvate of the compound represented by above formula 1, may exhibit a differential scanning calorimetry (DSC) endothermic transition peak at 154 to 161°C and 173 to 190°C when a heating rate is 10°C/min. Specifically, the crystal form G may have a DSC spectrum as shown in FIG. 12.

### Method for preparing crystal form G

The present invention may provide a method for preparing the crystal form G, which is a 1,4-dioxane solvate of the compound represented by above formula 1, the method including: 1) dissolving a compound represented by above formula 1 in 1,4-dioxane; and 2) slowly cooling down the solution obtained from above step 1) to room temperature and stirring for 1 to 24 hours.

In the case of the method for preparing the crystal form G, which is a 1,4-dioxane solvate of the compound represented by above formula 1 of the present invention, mass production may be possible with high yield and purity at low cost.

In one embodiment of the present invention, above step 1) may be a step of stirring under reflux. In this regard, the content may be the same as described in the method for preparing the crystal form A.

In one embodiment of the present invention, above step 2) may include a step of slowly cooling down the solution obtained from step 1) to room temperature. In this regard, the content may be the same as described in the method for preparing the crystal form A.

The method for preparing the crystal form G may further include step 3) below.

In one embodiment of the present invention, step 3) may further include a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be 1,4-dioxane.

In the present invention, the description about the term "room temperature" may be the same as described in the method for preparing the crystal form A.

The method for preparing the crystal form G may further include step 4) below.

In one embodiment of the present invention, step 4) may be a step of drying the washed solid obtained from step 3) at a temperature of 30 to 60°C, specifically hot air drying at a temperature of 35 to 55°C.

### Crystal form H

In another embodiment of the present invention, there may be provided a crystal form H of a compound represented by above formula 1 having a peak at diffraction angles (2θ±0.2°) of 7.48°, 10.24°, 10.35°, 11.10°, 13.55° and 15.18° in a powder X-ray diffraction analysis pattern.

In one embodiment of the present invention, the crystal form H of the compound represented by above formula 1 may be a tetrahydrofuran solvate of the compound represented by above formula 1. Specifically, the crystal form H may be a tetrahydrofuran solvate of the compound represented by above formula 1, and more specifically in a solvate of the compound represented by above formula 1, which is the crystal form H, a molar ratio between the compound represented by above formula 1 and tetrahydrofuran may be 1:1 to 3, specifically 1:1.

The crystal form H, which is a tetrahydrofuran solvate of the compound represented by above formula 1 of the present invention, may have high purity, may be safe, and may have excellent stability against heat and humidity, and thus may be stably maintained without any change in content in the long term. The same state may be maintained even after producing a preparation by using the crystal form H, which is a tetrahydrofuran solvate of the compound represented by above formula 1 of the present invention, or producing a preparation containing the same, and thus the content uniformity of the preparation may be stably maintained for a long period of time and may be easily applied to mass production.

In particular, the crystal form H, which is a tetrahydrofuran solvate of the compound represented by above formula 1 of the present invention, may exhibit non-hygroscopicity, and thus may be very advantageous for processing and storage of pharmaceuticals, and thus may be easily formulated into a preparation.

In addition, the crystal form H, which is a tetrahydrofuran solvate of the compound represented by above formula 1 of the present invention, may exhibit a high concentration in blood and thus have high bioavailability, thereby exhibiting an excellent pharmacological effect.

Thus, the crystal form H, which is a tetrahydrofuran solvate of the compound represented by formula 1 of the present invention, may be effectively used as a novel effective ingredient in a pharmaceutical composition capable of treating cancer diseases.

In one embodiment of the present invention, the crystal form H of the compound represented by above formula 1 may further include at least one peak at diffraction angles (2θ±0.2°) of 8.01°, 11.72°, 13.17°, 13.59°, 15.93°, 17.78°, 18.26°, 19.03°, 19.41°, 20.86°, 21.23°, 22.41°, 23.55°, 23.95°, 24.83°, 25.26°, 25.55°, 26.47°, 26.83° and 27.94° in a powder X-ray diffraction analysis pattern.

In one embodiment of the present invention, the crystal form H of the compound represented by above formula 1 may be one in which a powder X-ray diffraction analysis pattern is obtained by using CuKα radiation.

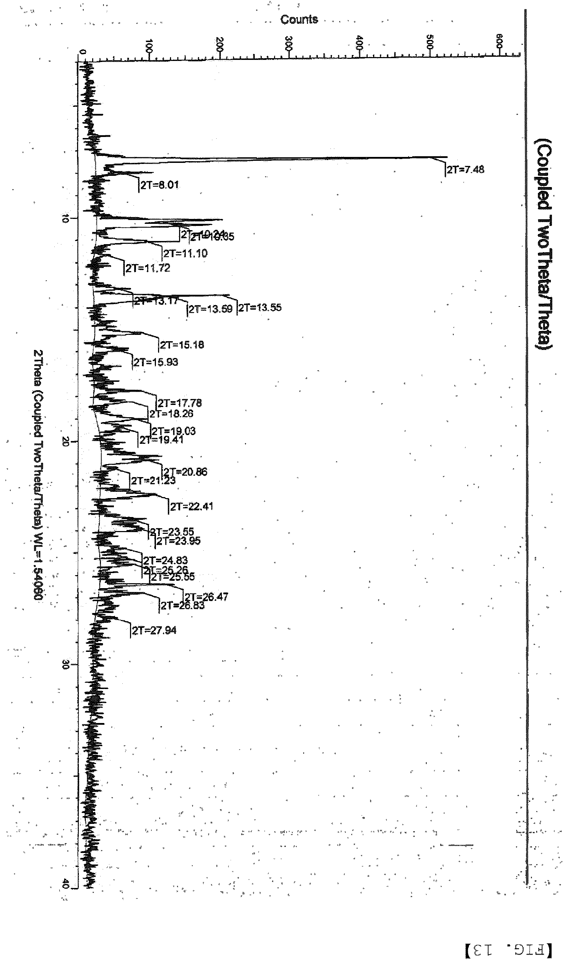

The crystal form H of the compound represented by above formula 1 of the present invention may have substantially the same spectrum as a powder X-ray diffraction (PXRD) analysis spectrum shown in FIG. 13.

The compound represented by above formula 1 may selectively inhibit PI3 kinase selected from the group consisting of PI3Kα, PI3Kβ, PI3Kδ and PI3Kγ.

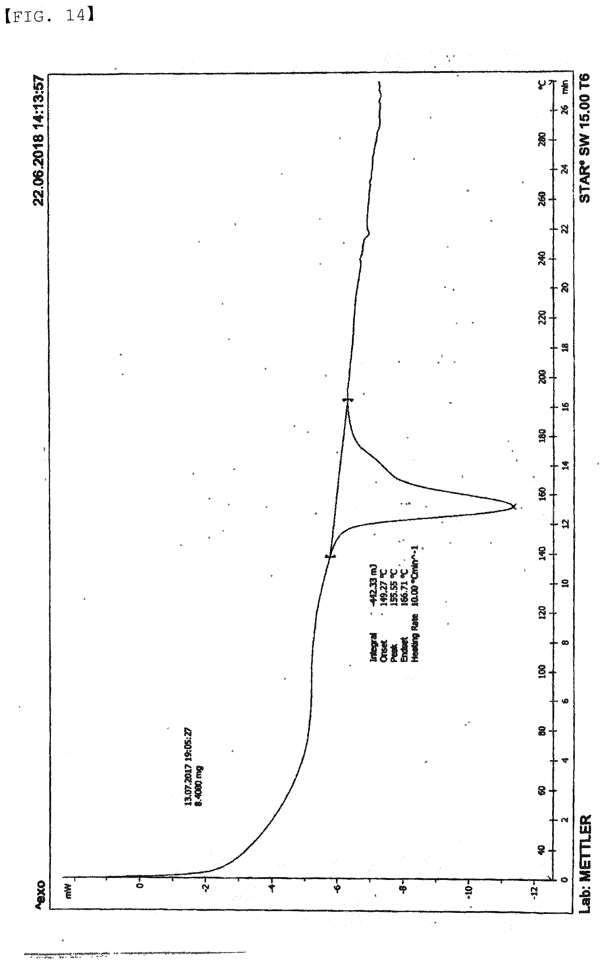

The crystal form H, which is a tetrahydrofuran solvate of the compound represented by above formula 1, may exhibit a differential scanning calorimetry (DSC) endothermic transition peak at 149 to 166°C when a heating rate is 10°C/min. Specifically, the crystal form H may have a DSC spectrum as shown in FIG. 14.

### Method for preparing crystal form H

The present invention may provide a method for preparing the crystal form H, which is a tetrahydrofuran solvate of the compound represented by above formula 1, the method including: 1) dissolving a compound represented by above formula 1 in tetrahydrofuran; and 2) slowly cooling down the solution obtained from above step 1) to room temperature and stirring for 1 to 24 hours.

In the case of the method for preparing the crystal form H, which is a tetrahydrofuran solvate of the compound represented by above formula 1 of the present invention, mass production may be possible with high yield and purity at low cost.

In one embodiment of the present invention, above step 1) may be a step of stirring under reflux. In this regard, the content may be the same as described in the method for preparing the crystal form A.

In one embodiment of the present invention, above step 2) may include a step of slowly cooling down the solution obtained from step 1) to room temperature. In this regard, the content may be the same as described in the method for preparing the crystal form A.

The method for preparing the crystal form H, which is a tetrahydrofuran solvate of the compound represented by above formula 1, may further include step 3) below.

In one embodiment of the present invention, step 3) may further include a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be tetrahydrofuran.

In the present invention, the description about the term "room temperature" may be the same as described in the method for preparing the crystal form A.

The method for preparing the crystal form H may further include step 4) below.

In one embodiment of the present invention, step 4) may be a step of drying the washed solid obtained from step 3) at a temperature of 30 to 60°C, specifically hot air drying at a temperature of 35 to 55°C.

### Crystal form I

The present invention may provide a crystal form I of a compound represented by above formula 1 having a peak at diffraction angles (2θ±0.2°) of 12.03°, 15.19°, 21.77°, 22.81°, 24.21° and 27.11° in a powder X-ray diffraction analysis pattern.

The crystal form I of the present invention may have high purity, may be safe, and may have excellent stability against heat and humidity, and thus may be stably maintained without any change in content in the long term. Thus, the same state may be maintained even after producing a preparation by using the crystal form I of the present invention or producing a preparation containing the same, and thus the content uniformity of the preparation may be stably maintained for a long period of time and may be easily applied to mass production.

In particular, the crystal form I may exhibit non-hygroscopicity, and thus may be very advantageous for processing and storage of pharmaceuticals, and thus may be easily formulated into a preparation.

In addition, the crystal form I of the present invention may exhibit an excellent pharmacological effect due to a high concentration in blood and thus may be effectively used as a novel effective ingredient in a pharmaceutical composition capable of treating cancer diseases, and the crystal form I of the present invention may show high bioavailability when orally administered, and thus may exhibit an excellent therapeutic effect even with a small amount of use, so that the form may remarkably improve the convenience of taking medication for patients.

Out of the crystal polymorphisms of the PI3K inhibitor, the crystal form I may show relatively high bioavailability and thus have an excellent therapeutic effect, so that the form may be safe, cause fewer side effects, and have an effect of remarkably improving the convenience of taking medication for patients.

In one embodiment of the present invention, the crystal form I of the compound represented by above formula 1 may further include at least one peak at diffraction angles (2θ±0.2°) of 7.56°, 9.84°, 10.08°, 10.56°, 10.83°, 11.84°, 12.41°, 12.87°, 13.54°, 14.52°, 14.75°, 15.90°, 16.45°, 16.90°, 17.86°, 18.17°, 18.55°, 18.95°, 19.62°, 20.39°, 20.99°, 22.04°, 22.43°, 22.99°, 23.55°, 24.36°, 25.26°, 25.78°, 27.31°, 27.79°, 28.88° and 30.83° in a powder X-ray diffraction analysis pattern.

In one embodiment of the present invention, the crystal form I of the compound represented by above formula 1 may be one in which a powder X-ray diffraction analysis pattern is obtained by using CuKα radiation.

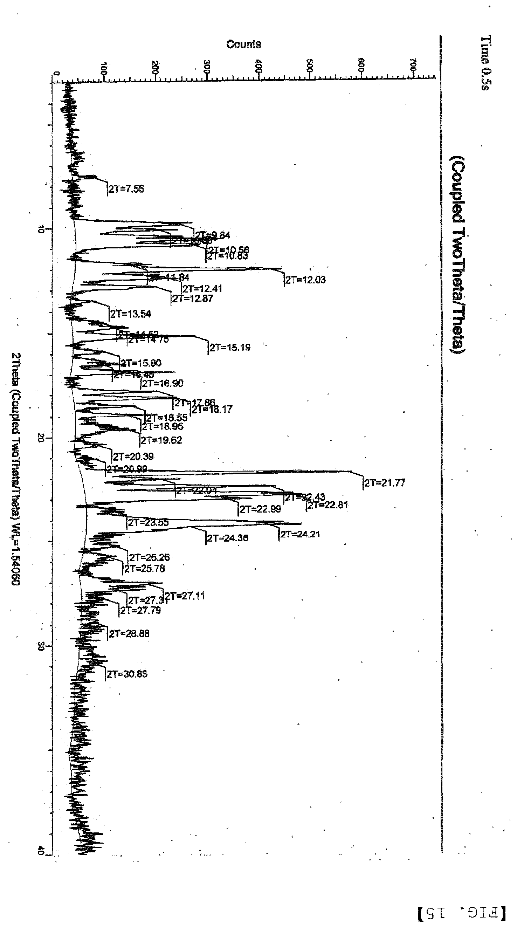

The crystal form I of the compound represented by above formula 1 of the present invention may have substantially the same spectrum as a powder X-ray diffraction (PXRD) analysis spectrum shown in FIG. 15.

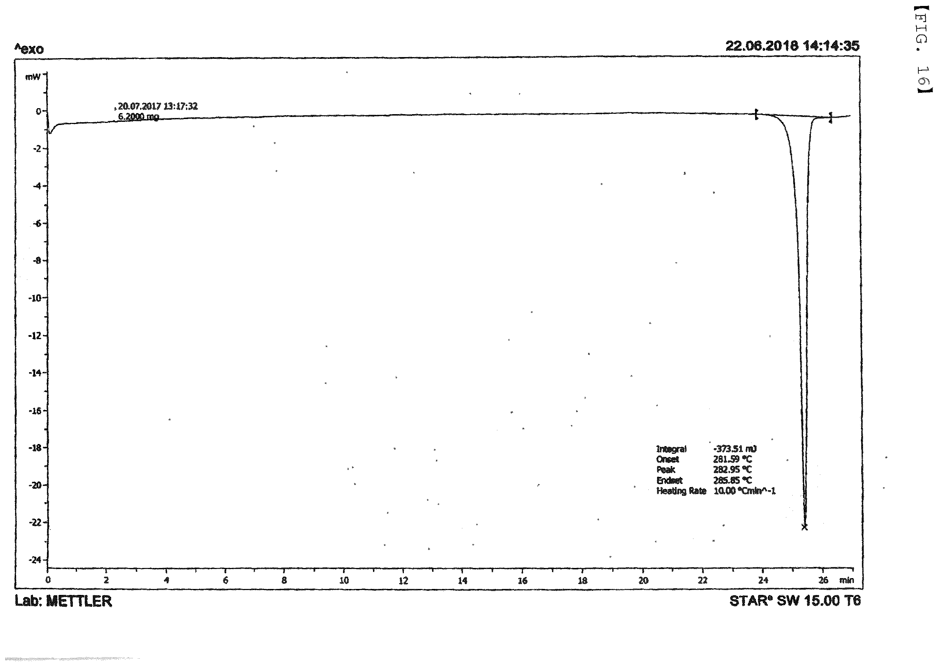

The crystal form I of the compound represented by above formula 1 may exhibit a differential scanning calorimetry (DSC) endothermic transition peak at 281 to 285°C when a heating rate is 10°C/min. Specifically, the crystal form I may have a DSC spectrum as shown in FIG. 16.

In one embodiment of the present invention, the crystal form I of the compound represented by formula 1 may be anhydride as a non-solvate.

### Method for preparing crystal form I

The present invention may provide a method for preparing the crystal form I, the method including: 1) adding a compound represented by above formula 1 into water, followed by refluxing; and 2) slowly cooling down the resulting solution to room temperature and stirring for 1 to 24 hours.

In the case of the method for preparing the crystal form I of the present invention, mass production may be possible with high yield and purity at low cost.

In one embodiment of the present invention, above step 1) may be a step of stirring under reflux. In this regard, the content may be the same as described in the method for preparing the crystal form A.

In one embodiment of the present invention, above step 2) may include a step of slowly cooling down the solution obtained from step 1) to room temperature. In this regard, the content may be the same as described in the method for preparing the crystal form A.

In the method for preparing the crystal form I, water was added to the compound represented by above formula 1, and thus the compound may be converted into the crystal form I by stirring under reflux in a state in which the compound is not dissolved in water.

The method for preparing the crystal form I may further include step 3) below.

In one embodiment of the present invention, step 3) may further include a step of filtering a solid obtained from step 2) and washing with any one solvent selected from the group consisting of ethanol, acetone, acetonitrile, 1,4-dioxane, tetrahydrofuran, water and a mixture thereof. Specifically, the solvent used in above step 3) may be purified water.

The method for preparing the crystal form H may further include step 4) below.

In one embodiment of the present invention, step 4) may be a step of drying the washed solid obtained from step 3) at a temperature of 30 to 60°C, specifically hot air drying at a temperature of 35 to 55°C.

### Pharmaceutical composition for preventing or treating cancer diseases, including crystal polymorphism

A pharmaceutical composition for preventing or treating cancer diseases of the present invention includes the crystal form A, crystal form B, crystal form C, crystal form D, crystal form E, crystal form G, crystal form H, crystal form I or a mixture thereof.

The pharmaceutical composition for preventing or treating cancer diseases of the present invention includes the crystal form A, crystal form B, crystal form C, crystal form D, crystal form E, crystal form G, crystal form H, crystal form I or a mixture thereof, thereby providing a remarkably preventive and therapeutic activity for cancer. In particular, out of the crystal polymorphisms of the PI3K inhibitor, the crystal form I may show relatively high bioavailability and thus have an excellent therapeutic effect, so that the form may be safe, cause fewer side effects, and have an effect of remarkably improving the convenience of taking medication for patients.

In the present invention, the term "prevention" refers to an inhibition or delay of occurrence of disease, disorder or condition. If the occurrence of disease, disorder or condition is inhibited or delayed for an expected period of time, the prevention may be considered as complete.

In the present invention, the term "treatment" refers to one which partially or completely reduces, ameliorates, alleviates, inhibits or delays a certain disease, disorder and/or condition, or symptoms of the condition, reduces a severity thereof, or reduces the occurrence of at least one symptom or property thereof.

In one embodiment of the present invention, the pharmaceutical composition of the present invention may be formulated into a preparation by using a pharmaceutically acceptable carrier according to a method easily practicable by those skilled in the art, to which the present invention pertains, such that the composition may be prepared in a unit dose form or prepared by being inserted into a multi-dose container.

In the present invention, the term "carrier" means a compound that facilitates the addition of a compound into a cell or tissue, and the term "pharmaceutically acceptable" refers to a composition which is physiologically acceptable and does not conventionally cause an allergic reaction such as gastrointestinal disturbance and dizziness, or other reactions similar thereto, when being administered into humans.

The pharmaceutically acceptable carrier is one which is conventionally used in formulating a preparation, including, but not limited thereto, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propylhydroxybenzoate, talc, magnesium stearate, mineral oil and the like.

The pharmaceutical composition of the present invention may further include additives such as fillers, anti-coagulants, lubricants, humectants, fragrance, emulsifiers, preservatives, etc. in addition to the above ingredients. In the present invention, a content of additives contained in the pharmaceutical composition is not particularly limited, and may be appropriately adjusted within a range of contents conventionally used in formulating a preparation.

The pharmaceutical composition of the present invention may be used for oral administration. In the present invention, the term "oral administration" means one, in which a substance prepared for allowing an active substance to be digested is administered into the gastrointestinal tract for absorption. A non-limiting example of the preparation for oral administration may include tablets, troches, lozenges, water soluble suspensions, oil suspensions, prepared powders, granules, emulsions, hard capsules, soft capsules, syrups, elixirs or the like. To formulate the pharmaceutical composition of the present invention into preparations for oral administration, the followings may be used: binders such as lactose, saccharose, sorbitol, mannitol, starch, amylopectin, cellulose, gelatin or the like; excipients such as dicalcium phosphate, etc.; disintegrants such as maize starch, sweet potato starch or the like; lubricants such as magnesium stearate, calcium stearate, sodium stearyl fumarate, polyethylene glycol wax, or the like; etc., in which sweetening agents, flavoring agents, syrups, etc. may also be used. Furthermore, in case of the capsules, liquid carriers such as fatty oil, etc. may be further used in addition to the above-mentioned materials.

The composition of the present invention may be used by being formulated into various forms such as an oral dosage form such as liquid, suspension, powder, granule, tablet, capsule, pill, extract, emulsion, syrup, aerosol, etc., an injectable agent of sterilized injectable solution, etc. in accordance with a conventional method for each purpose of use, in which the composition may be orally administered or administered through various routes including intravenous, intraperitoneal, subcutaneous, intrarectal, local administration, or the like.

A preferable dosage of the pharmaceutical composition of the present invention may vary in a range thereof depending on a patient's state, weight, age, gender, health condition, dietary and constitutional specificity, property of a preparation, a degree of disease, an administration time of the composition, an administration method, an administration period or interval, an excretion rate and a drug form, but may be appropriately selected by those skilled in the art. For example, the dosage may be in a range of about 0.1 to 1,000 mg/kg, in a range of about 1 to 800 mg/kg, in a range of about 5 to 600 mg/kg, or in a range of about 10 to 400 mg/kg, and may be specifically in a range of about 50 to 500 mg/kg, but not limited thereto, and may be administered once a month.

In the present specification, the term "effective dosage of a pharmaceutical composition" means an amount of the composition containing an active ingredient sufficient to treat a specific symptom. The dosage may vary depending on a method for formulating the pharmaceutical composition, an administration mode, an administration time and/or an administration route, etc., and may be diversified according to various factors including a type and degree of reaction to be achieved by administration of the pharmaceutical composition, a type of an individual for administration, the individual's age, weight, general health condition, disease symptom or severity, gender, diet and excretion, ingredients of other drug compositions to be used for the corresponding individual at the same time or different times, etc., as well as other similar factors well known in a pharmaceutical field, and those skilled in the art may easily determine and prescribe an effective dosage for intended treatment.

The pharmaceutical composition of the present invention may be administered once a day or several times a day by dividing the daily dosage of the composition. The pharmaceutical composition of the present invention may be administered as an individual therapeutic agent or in combination with other therapeutic agents, and may be administered sequentially or simultaneously with a conventional therapeutic agent. Considering all the above factors, the pharmaceutical composition of the present invention may be administered in such an amount that a maximum effect may be achieved by a minimum amount without a side effect, and such amount may be easily determined by those skilled in the art to which the present invention pertains.

In the present invention, the term "cancer" means all the conditions, in which a problem medically occurs to regulatory functions of cells for normal division, differentiation or death, then the cells are abnormally subjected to an excessive proliferation to infiltrate into surrounding tissues or organs, and then a mass of cells is formed to destroy or transform an existing structure. Cancer may be roughly classified into primary cancer present in a site of origin and metastatic cancer, which spreads from the site of origin to other parts of the human body.

In one embodiment of the present invention, the cancer disease may be any one of lung cancer, non-small cell lung cancer (NSCL), bronchiolo-alveolar cell lung cancer, gastric cancer, gastrointestinal cancer, liver cancer, bone cancer, pancreatic cancer, skin cancer, head and neck cancer, skin or ocular melanoma, uterine cancer, ovarian cancer, rectal cancer, colorectal cancer, colon cancer, breast cancer, sarcoma of uterus, opian tube carcinoma, internal endometrium carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penis cancer, prostate cancer, multiple myeloma, chronic or acute leukemia, solid tumor of childhood, lymphoma, bladder cancer, renal cancer, renal cell carcinoma, renal pelvic carcinoma, spinal axis tumor, brainstem glioma or pituitary gland adenoma.

The crystal polymorphisms included in the pharmaceutical composition for preventing or treating cancer diseases of the present invention are substantially the same as described for the crystal form A, crystal form B, crystal form C, crystal form D, crystal form E, crystal form G, crystal form H, and crystal form I.

### Method for preventing or treating cancer diseases

Other object of the present invention is to provide a method for preventing or treating cancer, the method including administering a pharmaceutical composition for preventing or treating cancer diseases, including the crystal form A, crystal form B, crystal form C, crystal form D, crystal form E, crystal form G, crystal form H, crystal form I or a mixture thereof into an subject in need.

In the present invention, the term "subject" means mammals such as monkey, cow, horse, dog, cat, rabbit, rat, mouse, etc., and in particular may include humans. The preventive or therapeutic method may include administering a therapeutically effective amount.

In the present invention, the term "therapeutically effective amount" refers to an amount of the crystal form A, crystal form B, crystal form C, crystal form D, crystal form E, crystal form G, crystal form H, crystal form I or a mixture thereof, which is effective in preventing or treating cancer.

The preventive or therapeutic method of the present invention includes not only dealing with the diseases per se before expression of their symptoms, but also inhibiting or avoiding such symptoms by administering the pharmaceutical composition for preventing or treating cancer diseases, containing the crystal form A, crystal form B, crystal form C, crystal form D, crystal form E, crystal form G, crystal form H, crystal form I or a mixture thereof. In managing the disease, a preventive or therapeutic dose of a certain active ingredient may vary depending on a nature and severity of the disease or condition and a route of administering the active ingredient. A dose and a frequency thereof may vary depending on an individual patient's age, weight and reactions. A suitable dose and usage may be easily selected by those skilled in the art, naturally considering such factors. In addition, the preventive or therapeutic method of the present invention may further include administering a therapeutically effective amount of an additional active agent, which is helpful in treating diseases, along with the pharmaceutical composition containing the crystal form A, crystal form B, crystal form C, crystal form D, crystal form E, crystal form G, crystal form H, crystal form I or a mixture thereof for preventing or treating cancer diseases, in which the additional active agent may achieve a synergy effect or an additive effect together with the pharmaceutical composition for preventing or treating cancer diseases, containing the crystal form A, crystal form B, crystal form C, crystal form D, crystal form E, crystal form G, crystal form H, crystal form I or a mixture thereof.

The crystal polymorphisms included in the pharmaceutical composition for preventing or treating cancer diseases of the present invention are substantially the same as described for the crystal form A, crystal form B, crystal form C, crystal form D, crystal form E, crystal form G, crystal form H, and crystal form I.

### Use in the manufacture of a medicament for preventing or treating cancer

An another object of the present invention is to provide a use of a pharmaceutical composition containing the crystal form A, crystal form B, crystal form C, crystal form D, crystal form E, crystal form G, crystal form H, crystal form I or a mixture thereof in the manufacture of medicament for preventing or treating cancer.

In one embodiment of the present invention, the pharmaceutical composition containing the crystal form A, crystal form B, crystal form C, crystal form D, crystal form E, crystal form G, crystal form H, crystal form I or a mixture thereof in the manufacture of medicament may be mixed with an acceptable carrier, etc. and further contain other agents.

The crystal polymorphisms included in the pharmaceutical composition for preventing or treating cancer diseases of the present invention are substantially the same as described for the crystal form A, crystal form B, crystal form C, crystal form D, crystal form E, crystal form G, crystal form H, and crystal form I.

Matters mentioned in the crystal polymorphisms, the method for preparing crystal polymorphisms, the pharmaceutical composition, the treatment method and the use of the present invention may be applied the same, if not contradictory to each other.

### [Advantageous Effects]

A novel crystal form of the P13K inhibitor of the present invention can be advantageous for being formulated into preparations due to low hygroscopicity, can be excellent in stability depending on accelerated, long-term and stress storage conditions, thermal stability and stability depending on pH, and thus can be stably maintained without any change in the content in the long term, and a novel crystalline compound can show an excellent pharmacological effect with a high concentration in blood and thus can be advantageous for a pharmaceutical preparation.

### [Brief Description Of The Drawings]

FIG. 1 is a view showing the results of powder X-ray diffraction (PXRD) analysis of the novel crystal form A according to Example 1 of the present invention (the unit of 2Theta on the horizontal axis is °).
FIG. 2 is a view showing the results of a differential scanning calorimetry (DSC) thermogram of the novel crystal form A according to Example 1 of the present invention.
FIG. 3 is a view showing the results of powder X-ray diffraction (PXRD) analysis of the novel crystal form B according to Example 2 of the present invention (the unit of 2Theta on the horizontal axis is °).
FIG. 4 is a view showing the results of a differential scanning calorimetry (DSC) thermogram of the novel crystal form B according to Example 3 of the present invention.
FIG. 5 is a view showing the results of powder X-ray diffraction (PXRD) analysis of the novel crystal form C according to Example 3 of the present invention (the unit of 2Theta on the horizontal axis is °).
FIG. 6 is a view showing the results of a differential scanning calorimetry (DSC) thermogram of the novel crystal form C according to Example 3 of the present invention.
FIG. 7 is a view showing the results of powder X-ray diffraction (PXRD) analysis of the novel crystal form D according to Example 4 of the present invention (the unit of 2Theta on the horizontal axis is °).
FIG. 8 is a view showing the results of a differential scanning calorimetry (DSC) thermogram of the novel crystal form D according to Example 4 of the present invention.
FIG. 9 is a view showing the results of powder X-ray diffraction (PXRD) analysis of the novel crystal form E according to Example 5 of the present invention (the unit of 2Theta on the horizontal axis is °).
FIG. 10 is a view showing the results of a differential scanning calorimetry (DSC) thermogram of the novel crystal form E according to Example 5 of the present invention.
FIG. 11 is a view showing the results of powder X-ray diffraction (PXRD) analysis of the novel crystal form G according to Example 6 of the present invention (the unit of 2Theta on the horizontal axis is °).
FIG. 12 is a view showing the results of a differential scanning calorimetry (DSC) thermogram of the novel crystal form G according to Example 6 of the present invention.
FIG. 13 is a view showing the results of powder X-ray diffraction (PXRD) analysis of the novel crystal form H according to Example 7 of the present invention (the unit of 2Theta on the horizontal axis is °).
FIG. 14 is a view showing the results of a differential scanning calorimetry (DSC) thermogram of the novel crystal form H according to Example 7 of the present invention.
FIG. 15 is a view showing the results of powder X-ray diffraction (PXRD) analysis of the novel crystal form I according to Example 8 of the present invention (the unit of 2Theta on the horizontal axis is °).
FIG. 16 is a view showing the results of a differential scanning calorimetry (DSC) thermogram of the novel crystal form I according to Example 8 of the present invention.

### [Mode for Invention]

Hereinafter, the present invention will be described in detail through Examples for better understanding of the present invention. However, the following Examples are provided only for the purpose of illustrating the present invention, and thus the scope of the present invention is not limited thereto. The Examples of the present invention are provided to more completely describe the present invention to those having ordinary skill in the art.

The reagents and solvents mentioned below were purchased from Sigma-Aldrich Korea, TCI, Alfa Aesar unless otherwise specified, and Agilent Technologies' 1100Series was used for the HLPC. ¹H NMR data were measured by using a Bruker Avance III (400 MHz).

In addition, various crystals of (S)-4-((1-(4,8-dichloro-1-oxo-2-phenyl-1,2-dihydroisoquinoline-3-yl)ethyl)amino)pyrido[2,3-d]pyrimidin-5(8H)-one prepared by Examples below were confirmed with an X-ray diffraction pattern by an X-ray diffraction method and a DSC pattern by differential scanning calorimetry (DSC) method. The X-ray diffraction analysis used in the following examples was performed using a BRUKER AXS powder X-ray diffractometer model D2 PHASER equipped with a rotation measuring instrument and a solid state detector, and a measurement was made by a known method in the art using Cu-Kα rays in an analysis range of 0° to 40° at an irradiation rate of 3° per minute. In addition, the DSC measurement performed by the differential scanning calorimetry (DSC) method shows a value measured at 30-300°C with HP DSC1 of METTLER TOREDO while raising a temperature at a rate of 10°C/min.

### Preparation Example 1. Preparation of (S)-4-((1-(4,8-dichloro-1-oxo-2-phenyl-1,2-dihydroisoquinoline-3-yl)ethyl)amino)pyrido[2,3-d]pyrimidine-5(8H)-one

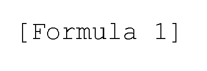

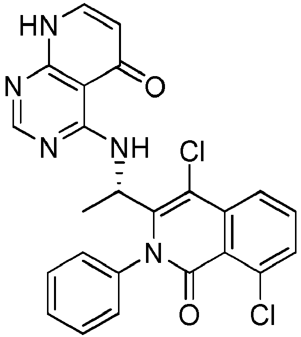

The title compound of (S)-4-((1-(4,8-dichloro-1-oxo-2-phenyl-1,2-dihydroisoquinoline-3-yl)ethyl)amino)pyrido[2,3-d]pyrimidin-5(8H)-one was prepared by the same method as described in Example 10 of the International Publication WO2016/204429.

### Example 1. Crystal form A

The 6 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 240 mL of ethanol, dissolved while stirring under reflux, slowly cooled down to room temperature, and stirred overnight. The resulting solid was filtered, washed with 30 mL of ethanol, and dried in a hot air dryer at 45°C, so as to obtain 4.12 g of a crystal form A compound (yield of 69%).

With respect to the solid compound, a powder X-ray diffraction (PXRD) analysis peak and a differential scanning calorimetry (DSC) endothermic peak were analyzed and the results thereof are shown in FIGS. 1 and 2, respectively.

As confirmed in FIG. 1, the solid compound exhibited a peak at 2θ (±0.2°) values of 6.71°, 7.36°, 9.39°, 12.34°' 20.76° and 24.13°.

In addition, as confirmed in FIG. 2, it can be seen that the solid compound exhibits a differential scanning calorimetry (DSC) endothermic transition peak at 178 to 185°C when a heating rate is 10°C/min.

### Example 2. Crystal form B

The 4 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 200 mL of acetone, dissolved while stirring under reflux, slowly cooled down to room temperature, and stirred overnight. The resulting solid was filtered, washed with 15 mL of acetone, and dried in a hot air dryer at 45°C, so as to obtain 1.61 g of a crystal form B compound (yield of 40%).

With respect to the solid compound, a powder X-ray diffraction (PXRD) analysis peak and a differential scanning calorimetry (DSC) endothermic peak were analyzed and the results thereof are shown in FIGS. 3 and 4, respectively.

As confirmed in FIG. 3, the solid compound exhibited a peak at 2θ (±0.2°) values of 3.34°, 6.20°, 7.06°, 9.45°' 18.82° and 24.97°.

In addition, as confirmed in FIG. 4, it can be seen that the solid compound exhibits a differential scanning calorimetry (DSC) endothermic transition peak at 187 to 194°C when a heating rate is 10°C/min.

### Example 3. Crystal form C

The 6 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 240 mL of ethanol and dissolved while stirring under reflux, after which 240 mL of purified water (H₂O) was added dropwise, slowly cooled down to room temperature, and stirred overnight. The resulting solid was filtered, washed with 30 mL of ethanol, and dried in a hot air dryer at 45°C, so as to obtain 5.15 g of a crystal form C compound (yield of 86%).

With respect to the solid compound, a powder X-ray diffraction (PXRD) peak and a differential scanning calorimetry (DSC) endothermic peak were analyzed and the results thereof are shown in FIGS. 5 and 6, respectively.

As confirmed in FIG. 5, the solid compound exhibited a peak at 2θ (±0.2°) values of 3.72°, 6.27°, 7.22°, 9.58°' 18.90° and 25.14°.

In addition, as confirmed in FIG. 6, it can be seen that the solid compound exhibits a differential scanning calorimetry (DSC) endothermic transition peak at 186 to 192°C when a heating rate is 10°C/min.

### Example 4. Crystal form D

The 6 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 300 mL of acetonitrile, dissolved while stirring under reflux, slowly cooled down to room temperature, and stirred overnight. The resulting solid was filtered, washed with 24 mL of acetonitrile, and dried in a hot air dryer at 45°C, so as to obtain 4.86 g of a crystal form D compound (yield of 81%).

With respect to the solid compound, a powder X-ray diffraction (PXRD) analysis peak and a differential scanning calorimetry (DSC) endothermic peak were analyzed and the results thereof are shown in FIGS. 7 and 8, respectively.

As confirmed in FIG. 7, the solid compound exhibited a peak at 2θ (±0.2°) values of 3.77°, 6.14°, 6.88°, 7.51°' 9.25° and 9.92°.

In addition, as confirmed in FIG. 8, it can be seen that the solid compound exhibits a differential scanning calorimetry (DSC) endothermic transition peak at 185 to 194°C when a heating rate is 10°C/min.

### Example 5. Crystal form E

The 6 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 300 mL of acetonitrile and dissolved while stirring under reflux, after which 300 mL of purified water (H₂O) was added dropwise, slowly cooled down to room temperature, and stirred overnight. The resulting solid was filtered, washed with 24 mL of acetonitrile, and dried in a hot air dryer at 45°C, so as to obtain 5.12 g of a crystal form E compound (yield of 85%).

With respect to the solid compound, a powder X-ray diffraction (PXRD) analysis peak and a differential scanning calorimetry (DSC) endothermic peak were analyzed and the results thereof are shown in FIGS. 9 and 10, respectively.

As confirmed in FIG. 9, the solid compound exhibited a peak at 2θ (±0.2°) values of 3.81°, 6.25°, 6.98°, 7.61°' 9.35° and 26.74°.

In addition, as confirmed in FIG. 10, it can be seen that the solid compound exhibits a differential scanning calorimetry (DSC) endothermic transition peak at 196 to 211°C when a heating rate is 10°C/min.

### Example 6. Crystal form G

The 3 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 19.2 mL of 1,4-dioxane, dissolved while stirring under reflux, slowly cooled down to room temperature, and stirred overnight. The resulting solid was filtered, washed with 15 mL of 1,4-dioxane, and dried in a hot air dryer at 45°C, so as to obtain 2.56 g of a crystal form G compound (yield of 85%).

With respect to the solid compound, a powder X-ray diffraction (PXRD) analysis peak and a differential scanning calorimetry (DSC) endothermic peak were analyzed and the results thereof are shown in FIGS. 11 and 12, respectively.

As confirmed in FIG. 11, the solid compound exhibited a peak at 2θ (±0.2°) values of 7.33°, 8.56°, 9.69°, 9.94°' 19.97° and 25.37°.

In addition, as confirmed in FIG. 12, it can be seen that the solid compound exhibits a differential scanning calorimetry (DSC) endothermic transition peak at 154 to 161°C and 173 to 190°C when a heating rate is 10°C/min. An exothermic peak appearing after two endothermic peaks (melting) means that crystals have been precipitated. After crystal precipitation (exothermic peak), an endothermic peak again appeared at 281-285°C.

### Example 7. Crystal form H

The 3 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 35 mL of tetrahydrofuran (THF), dissolved while stirring under reflux, slowly cooled down to room temperature, and stirred overnight. The resulting solid was filtered, washed with 15 mL of tetrahydrofuran, and dried in a hot air dryer at 45°C, so as to obtain 1.26 g of a crystal form H compound (yield of 42%).

With respect to the solid compound, a powder X-ray diffraction (PXRD) analysis peak and a differential scanning calorimetry (DSC) endothermic peak were analyzed and the results thereof are shown in FIGS. 13 and 14, respectively.

As confirmed in FIG. 13, the solid compound exhibited a peak at 2θ (±0.2°) values of 7.48°, 10.24°, 10.35°, 11.10°' 13.55° and 15.18_{°}.

In addition, as confirmed in FIG. 14, it can be seen that the solid compound exhibits a differential scanning calorimetry (DSC) endothermic transition peak at 149 to 166°C when a heating rate is 10°C/min.

### Example 8. Crystal form I

The 2 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 20 mL of purified water, stirred under reflux for three hours, slowly cooled down to room temperature, and stirred overnight. The resulting solid was filtered, washed with 10 mL of purified water, and dried in a hot air dryer at 45°C, so as to obtain 1.94 g of a crystal form I compound (yield of 97%).

With respect to the solid compound, a powder X-ray diffraction (PXRD) analysis peak and a differential scanning calorimetry (DSC) endothermic peak were analyzed and the results thereof are shown in FIGS. 15 and 16, respectively.

As confirmed in FIG. 15, the solid compound exhibited a peak at 2θ (±0.2°) values of 12.03°, 15.19°, 21.77°, 22.81°' 24.21° and 27.11°.

In addition, as confirmed in FIG. 16, it can be seen that the solid compound exhibits a differential scanning calorimetry (DSC) endothermic transition peak at 281 to 285°C when a heating rate is 10°C/min.

### Example 9. Crystal form I

The 900 g of (S)-4-((1-(4,8-dichloro-1-oxo-2-phenyl-1,2-dihydroisoquinoline-3-yl)ethyl)amino)pyrido[2,3-d]pyrimidin-5(8H)-one) prepared in Preparation Example 1 was added to 4500 mL of purified water, stirred under reflux for three hours, slowly cooled down to room temperature, and stirred overnight. The resulting solid was filtered, washed with 2000 mL of purified water, and dried in a hot air dryer at 45°C, so as to obtain 881 g of a crystal form I compound (yield of 98%).

With respect to the solid compound, a powder X-ray diffraction (PXRD) analysis peak and a differential scanning calorimetry (DSC) endothermic peak were analyzed and the results thereof are shown in FIGS. 15 and 16, respectively.

### Experimental Example 1. Hygroscopicity (Dynamic vapor sorption, DVS) measurement experiment

The hygroscopicity was tested by using the crystal form I of above Example 8.
**a. Instruments used for experiment**
   i) Model name: DVS Intrinsic (Surface Measurement Systems LTD. UK)
   ii) Specifications
      Temperature: 25°C
      Temperature stability: ± 0.1°C
      Maximum sample mass: 1g/4g (low/high mass instrument)
      Mass change: ±150mg/1.0g (low/high mass instrument)
      Humidity range: 0 ~ 98% RH
      RH Accuracy: ±1% RH
      Typical gas (Nitrogen + water vapor) flow rate: 200sccm
      Sample chamber: 40mm wide × 50mm deep × 50mm high
      Heating system: Peltier + Cartridge
**b. Sample information**
   An experiment was performed on the crystal form I of above Example 8.
**c. Experimental conditions**
   i) Temperature: 25°C
   ii) Humidity: Initial 0% maximum 95%
   iii) Experiment time: 7 hours
   iv) Solvent: Water
**d. Experimental method**
   After placing a certain amount (10 mg) of the crystal form I compound prepared in Example 8 on a sample pan in a sample chamber, nitrogen was flowed through a tube connected into the sample chamber for the first one hour so as to remove moisture in the chamber and moisture of the sample. Humidity in the chamber was controlled by controlling water vapor, and target relative humidity was increased step by step from initial 0% to 10% every 20 minutes, so as to measure a moisture content of crystal form I for 205 min. A mass of the sample was measured every hour (from 0% RH to 95% RH) to collect data at 10% RH intervals, and a change in weight of the sample according to the increase and decrease of humidity was graphed.

A change in the water content of the crystal form I compound according to Example 8 is shown in table 1 below.

**[Table 1]**

| crystal polymorphism Relative humidity (%) | Crystal form I |
|---|---|
| 0.0 | 0.01 |
| 10.0 | 0.06 |
| 20.0 | 0.11 |
| 30.0 | 0.16 |
| 40.0 | 0.20 |
| 50.0 | 0.24 |
| 60.0 | 0.28 |
| 70.0 | 0.33 |
| 80.0 | 0.38 |
| 90.0 | 0.46 |
| 95.0 | 0.54 |
| | non-hygroscopicity |

As a result, as shown in above table 1, it was observed that the crystal form I compound exhibits almost no change in moisture content and thus has remarkably excellent non-hygroscopicity. This indicates that the crystal form I shows pharmaceutically remarkable excellence in all processes such as preparation, storage, formulation, etc., of the active ingredient.

Hereinafter, further experiments were performed on the crystal form I.

### Experimental Example 2. Pharmacokinetic (PK) test

A pharmacokinetic (PK) experiment was performed on the crystal form I compound according to Example 8.

The powder of the crystal form I compound of Example 8 was filled into a capsule in an amount of 100 mg, and the capsule was orally administered to a beagle dog at 100 mg once a day, and then the biokinetics of the drug was confirmed.

Specifically, 100 mg of the crystal form I compound was orally administered to the beagle dog, and blood was collected at a predetermined time, so as to measure a concentration of (S)-4-((1-(4,8-dichloro-1-oxo-2-phenyl-1,2-dihydroisoquinoline-3-yl)ethyl)amino)pyrido[2,3-d]pyrimidine-5(8H)-one. With respect to pharmacokinetic parameters, the BA Calc 2007 program was used to calculate an area under the plasma concentration-time curve from dosing time to final plasma concentration quantification time t (AUCₜ), area under the plasma concentration-time curve from dosing time to infinite time (AUCᵢ) , peak plasma concentration (Cₘₐₓ), time for maximum plasma concentration (Tₘₐₓ), and terminal elimination half-life (t_{1/2}). As a result, a significance between substances was confirmed by Student t-test (SPSS) at a 95% confidence interval, and the results thereof are shown in table 2 below.

**[Table 2]**

| PK Parameter | Crystal form I |
|---|---|
| t_{1/2} (hr) | 1.56 |
| Tₘₐₓ (hr) | 1.25 |
| Cmax (ng/mL) | 214.89 |
| AUCₗₐₛₜ (ng·hr/mL) | 540.14 |
| AUC_{INF} (ng·hr/mL) | 558.69 |

As can be confirmed in above table 2, the crystal form I compound of Example 8 exhibited a high concentration in blood.

Thus, it could be seen that the crystal form I compound according to Example 8 exhibits excellent bioavailability and thus has a remarkably excellent therapeutic effect.

### Experimental Example 3. Accelerated stability test

The powder of the crystal form I compound according to above Example 8 was doubly packed with an LDPE bag, then packed with a paperboard tube, and left alone at 40±2°C and RH 75±5% for zero and six months, after which impurities were measured by using a high speed liquid chromatography under the following conditions. The results thereof are shown in table 3 below.

### <HPLC conditions>

- Detector: Ultraviolet absorption spectrophotometer (wavelength: 307 nm)
- Column: Kromasil 100-5C18 (4.6 × 250 mm, 5 *µ*m) or a column similar thereto
- Column temperature: 25°C
- Injection amount: 10 µl
- Mobile phase: Mobile phase A - 20 mmol/L of ammonium acetate buffer solution (pH 4.5, acetic acid), Mobile phase B - acetonitrile:methanol=8:2

| | Mobile phase A (%) | Mobile phase A (%) | Flow rate (mL/min) |
|---|---|---|---|
| 0 | 75 | 25 | 1.0 |
| 5 | 75 | 25 | 1.0 |
| 35 | 30 | 70 | 1.0 |
| 50 | 30 | 70 | 1.0 |
| 51 | 75 | 25 | 1.0 |
| 60 | 75 | 25 | 1.0 |

**[Table 3]**

| Test item | Example 8 | |
|---|---|---|
| | Zero month (Initial) | Six months |
| Individual impurity (%) | 0.02 | 0.02 |
| Total impurity (%) | 0.14 | 0.16 |
| Content (%) | 100.0 | 99.7 |
| Crystal form | Form I | Form I |

| | | |
|---|---|---|
| (Unit: wt%) | | |

In the results of above table 3, with respect to the crystal form I compound of Example 8, neither significant change nor change with time was observed in terms of impurities and content.

Thus, it could be seen that the crystal form I compound of Example 8 may stably maintain high purity for six months under the accelerated conditions so as to provide excellent stability and a remarkably less occurrence of impurities, thereby maintaining excellent safety for a long period of time with almost no change in moisture content and with remarkably low hygroscopicity.

### Experimental Example 4. Long-term stability test

The powder of the crystal form I compound according to above Example 8 was doubly packed with an LDPE bag, then packed with a paperboard tube, and filled into a capsule, after which the capsule was subjected to PTP packing to carry out a long-term stability test (25±2°C and RH 60±5%). The results thereof are shown in table 4 below.

**[Table 4]**

| Test item | Example 8 | |
|---|---|---|
| | Zero month (Initial) | Six months |
| Individual impurity (%) | 0.02 | 0.02 |
| Total impurity (%) | 0.14 | 0.15 |
| Content (%) | 100.0 | 99.7 |
| Crystal form | Form I | Form I |

| | | |
|---|---|---|
| (Unit: wt%) | | |

In the results of above table 4, with respect to the crystal form I compound of Example 8, neither significant change nor change with time was observed in terms of impurities and content.

Thus, it could be seen that the crystal form I compound of Example 8 may stably maintain high purity for six months so as to provide excellent stability and a remarkably less occurrence of impurities, thereby maintaining excellent safety for a long period of time with almost no change in moisture content and with remarkably low hygroscopicity.

### Experimental Example 5. Stress stability test

The crystal form I prepared in above Example 8 was left alone under the stress conditions (temperature: 60°C, humidity: 40°°C, RH75%) for 0, 7 and 14 days, after which impurities were measured by using high speed liquid chromatography. The results thereof are shown in table 5 below.

**[Table 5]**

| Stress condition | Test item | Example 8 | | |
|---|---|---|---|---|
| | | Zero day (Initial) | 7 days | 14 days |
| Temperature (60°C) | Individual impurity (%) | 0.58 | 0.58 | 0.58 |
| | Total impurity (%) | 0.66 | 0.64 | 0.65 |
| | Content (%) | 99.1 | 100.0 | 99.7 |
| Humidity (40°C, RH75%) | Individual impurity (%) | 0.58 | 0.58 | 0.58 |
| | Total impurity (%) | 0.66 | 0.64 | 0.65 |
| | Content (%) | 99.1 | 99.6 | 100.8 |

| | | | | |
|---|---|---|---|---|
| (Unit: wt%) | | | | |

In the results of above table 5, with respect to the crystal form I compound of Example 8, neither significant change nor change with time was observed in terms of impurities and content.

Thus, it could be seen that the crystal form I compound of Example 8 may stably maintain high purity for six months so as to provide excellent stability and a remarkably less occurrence of impurities, thereby maintaining excellent safety for a long period of time with almost no change in moisture content and with remarkably low hygroscopicity.

## Claims

1. A crystal form A of a compound represented by formula 1 below, having a peak at diffraction angles (2θ±0.2°) of 6.71°, 7.36°, 9.39°, 12.34°, 20.76° and 24.13° in a powder X-ray diffraction pattern:

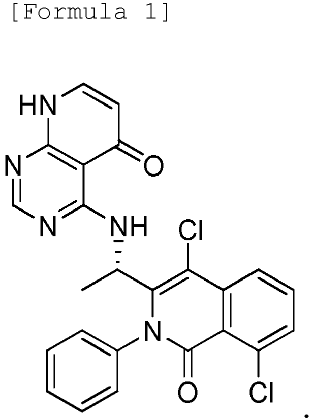

2. The crystal form A according to claim 1, wherein the crystal form A of the compound represented by above formula 1 further has at least one peak at diffraction angles (2θ±0.2°) of 5.75°, 8.19°, 9.87°, 11.12°, 13.71°, 16.12°, 17.88°, 18.90°, 19.71°, 23.21°, 25.18° and 27.09_{°} in a powder X-ray diffraction pattern.

3. The crystal form A according to claim 1, wherein the crystal form A has a differential scanning calorimetry (DSC) endothermic transition peak at 178 to 185°C when a heating rate is 10°C/min.

4. A method for preparing the crystal form A according to claim 1, the method comprising:
1) dissolving the compound represented by formula 1 according to claim 1 in C₁-C₄ alcohol; and
2) slowly cooling down the solution obtained from above step 1) to room temperature and stirring for 1 to 24 hours.

5. A crystal form B of a compound represented by formula 1 below, having a peak at diffraction angles (2θ±0.2°) of 3.34°, 6.20°, 7.06°, 9.45°, 18.82° and 24.97° in a powder X-ray diffraction pattern:

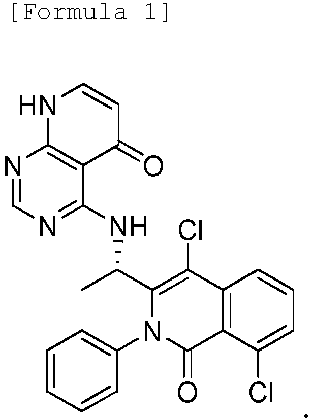

6. The crystal form B according to claim 5, wherein the crystal form B of the compound represented by above formula 1 further has at least one peak at diffraction angles (2θ±0.2°) of 7.33°, 8.06°, 8.25°, 13.89°, 16.22° and 26.42° in a powder X-ray diffraction pattern.

7. The crystal form B according to claim 5, wherein the crystal form B has a differential scanning calorimetry (DSC) endothermic transition peak at 187 to 194°C when a heating rate is 10°C/min.

8. A method for preparing the crystal form B of claim 5, the method comprising:
1) dissolving the compound represented by formula 1 according to claim 5 in C₃-C₁₀ ketone; and
2) slowly cooling down the solution obtained from above step 1) to room temperature and stirring for 1 to 24 hours.

9. A crystal form C of a compound represented by formula 1 below, having a peak at diffraction angles (2θ±0.2°) of 3.72°, 6.27°, 7.22°, 9.58°, 18.90° and 25.14° in a powder X-ray diffraction pattern:

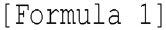

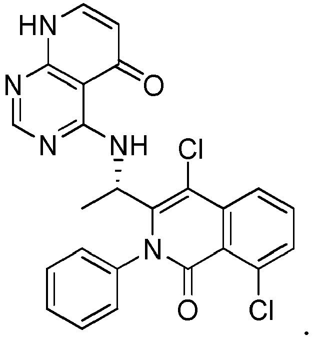

10. The crystal form C according to claim 9, wherein the crystal form C of the compound represented by above formula 1 further has at least one peak at diffraction angles (29±0.2°) of 5.11°, 7.42°, 8.09°, 10.15°, 13.08°, 16.37°,and 26.53° and 31.43° in a powder X-ray diffraction pattern.

11. The crystal form C according to claim 9, wherein the crystal form C has a differential scanning calorimetry (DSC) endothermic transition peak at 186 to 192°C when a heating rate is 10°C/min.

12. A method for preparing the crystal form C according to claim 9, the method comprising:
1) dissolving the compound represented by formula 1 according to claim 9 in C₁-C₄ alcohol;
2) adding water dropwise to the solution obtained from above step 1); and
3) slowly cooling down to room temperature and stirring for 1 to 24 hours.

13. A crystal form D of a compound represented by formula 1 below, having a peak at diffraction angles (29±0.2°) of 3.77°, 6.14°, 6.88°, 7.51°, 9.25° and 9.92° in a powder X-ray diffraction pattern:

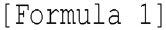

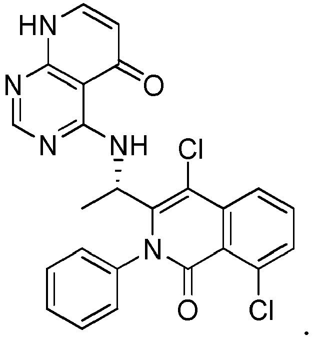

14. The crystal form D according to claim 13, wherein the crystal form D of the compound represented by above formula 1 further has at least one peak at diffraction angles (29±0.2°) of 10.57°, 12.37°, 12.69°, 14.09°, 14.79°, 15.75°, 17.18°, 18.13°, 18.85°, 19.61°, 20.22°, 20.99°, 23.39°, 23.87°, 24.61°, 26.42°, 27.63°, 29.76° and 30.31° in a powder X-ray diffraction pattern.

15. The crystal form D according to claim 13, wherein the crystal form D has a differential scanning calorimetry (DSC) endothermic transition peak at 185 to 194°C when a heating rate is 10°C/min.

16. A method for preparing the crystal form D according to claim 13, the method comprising:
1) dissolving the compound represented by formula 1 according to claim 13 in C₁-C₆ nitrile; and
2) slowly cooling down the solution obtained from above step 1) to room temperature and stirring for 1 to 24 hours.

17. A crystal form E of a compound represented by formula 1 below, having a peak at diffraction angles (29±0.2°) of 3.81°, 6.25°, 6.98°, 7.61°, 9.35° and 26.74° in a powder X-ray diffraction pattern.

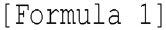

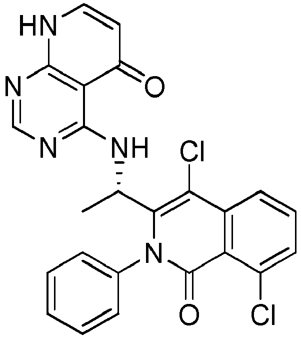

18. The crystal form E according to claim 17, wherein the crystal form E of the compound represented by above formula 1 further has at least one peak at diffraction angles (29±0.2°) of 10.29°, 10.67°, 12.04°, 12.36°, 13.51°, 14.28°, 14.76°, 15.16°, 15.87°, 16.28°, 16.98°, 17.89°, 18.71°, 19.47°, 19.77°, 20.66°, 21.07°, 22.56°, 23.04°, 23.56°, 24.98°, 25.39°, 25.77°, 27.11°, 28.08°, 28.34° and 29.24° in a powder X-ray diffraction pattern.

19. The crystal form E according to claim 17, wherein the crystal form E has a differential scanning calorimetry (DSC) endothermic transition peak at 196 to 211°C when a heating rate is 10°C/min.

20. A method for preparing the crystal form E according to claim 17, the method comprising:
1) dissolving the compound represented by formula 1 according to claim 17 in C₁-C₆ nitrile;
2) adding water dropwise to the solution obtained from above step 1); and
3) slowly cooling down to room temperature and stirring for 1 to 24 hours.

21. A crystal form G of a compound represented by formula 1 below, having a peak at diffraction angles (29±0.2°) of 7.33°, 8.56°, 9.69°, 9.94°, 19.97° and 25.37° in a powder X-ray diffraction pattern:

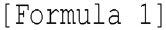

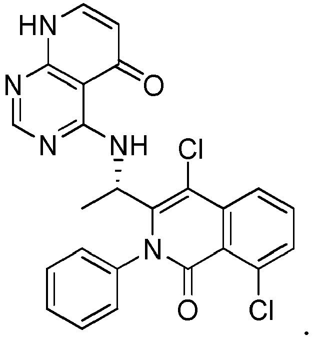

22. The crystal form G according to claim 21, wherein the crystal form G of the compound represented by above formula 1 further has at least one peak at diffraction angles (29±0.2°) of 4.94°, 13.34° and 15.53° in a powder X-ray diffraction pattern.

23. The crystal form G according to claim 21, wherein the crystal form G has a differential scanning calorimetry (DSC) endothermic transition peak at 154 to 161°C and 173 to 190°C when a heating rate is 10°C/min.

24. The crystal form G according to claim 21, wherein the crystal form G is a solvate of 1,4-dioxane.

25. A method for preparing the crystal form G according to claim 21, the method comprising:
1) dissolving the compound represented by formula 1 according to claim 21 in 1,4-dioxane; and
2) slowly cooling down the solution obtained from above step 1) to room temperature and stirring for 1 to 24 hours.

26. A crystal form H of a compound represented by formula 1 below, having a peak at diffraction angles (29±0.2°) of 7.48°, 10.24°, 10.35°, 11.10°, 13.55° and 15.18° in a powder X-ray diffraction pattern:

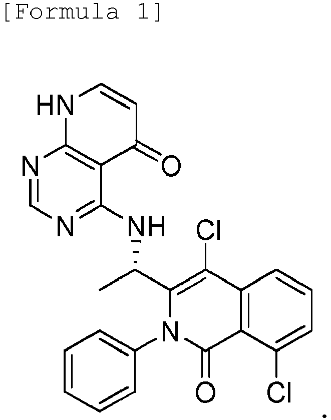

27. The crystal form H according to claim 26, wherein the crystal form H of the compound represented by above formula 1 further has at least one peak at diffraction angles (2θ±0.2°) of 8.01°, 11.72°, 13.17°, 13.59°, 15.93°, 17.78°, 18.26°, 19.03°, 19.41°, 20.86°, 21.23°, 22.41°, 23.55°, 23.95°, 24.83°, 25.26°, 25.55°, 26.47°, 26.83° and 27.94° in a powder X-ray diffraction pattern.

28. The crystal form H according to claim 26, wherein the crystal form H comprises a differential scanning calorimetry (DSC) endothermic transition peak at 149 to 166°C when a heating rate is 10°C/min.

29. The crystal form H according to claim 26, wherein the crystal form H is a solvate of tetrahydrofuran.

30. A method for preparing the crystal form H according to claim 26, the method comprising:
1) dissolving the compound represented by formula 1 according to claim 26 in tetrahydrofuran; and
2) slowly cooling down the solution obtained from above step 1) to room temperature and stirring for 1 to 24 hours.

31. A crystal form I of a compound represented by formula 1 below, having a peak at diffraction angles (29±0.2°) of 12.03°, 15.19°, 21.77°, 22.81°, 24.21° and 27.11° in a powder X-ray diffraction pattern:

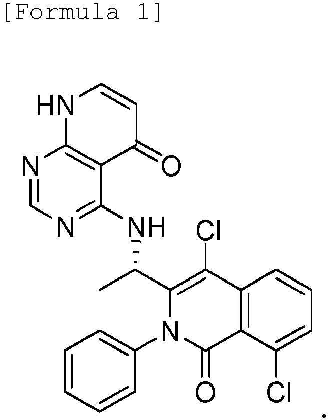

32. The crystal form I according to claim 31, wherein the crystal form I of the compound represented by above formula 1 further has at least one peak at diffraction angles (29±0.2°) of 7.56°, 9.84°, 10.08°, 10.56°, 10.83°, 11.84°, 12.41°, 12.87°, 13.54°, 14.52°, 14.75°, 15.90°, 16.45°, 16.90°, 17.86°, 18.17°, 18.55°, 18.95°, 19.62°, 20.39°, 20.99°, 22.04°, 22.43°, 22.99°, 23.55°, 24.36°, 25.26°, 25.78°, 27.31°, 27.79°, 28.88° and 30.83° in a powder X-ray diffraction pattern.

33. The crystal form I according to claim 31, wherein the crystal form I has a differential scanning calorimetry (DSC) endothermic transition peak at 281 to 285°C when a heating rate is 10°C/min.

34. A method for preparing the crystal form I according to claim 31, the method comprising:
1) adding the compound represented by formula 1 according to claim 31 to water, followed by refluxing; and
2) slowly cooling down to room temperature and stirring for 1 to 24 hours.

35. A pharmaceutical composition for treating or preventing cancer diseases, comprising the crystal form A of any one according to claims 1 to 3; the crystal form B of any one according to claims 5 to 7; the crystal form C of any one according to claims 9 to 11; the crystal form D of any one according to claims 13 to 15; the crystal form E of any one according to claims 17 to 19; the crystal form G of any one according to claims 21 to 24; the crystal form H of any one according to claims 26 to 29; or the crystal form I of any one according to claims 31 to 33; and a pharmaceutically acceptable carrier.
